(19) **Europäisches Patentamt / European Patent Office / Office européen des brevets**

(11) **EP 1 864 670 A1**

(12) **EUROPEAN PATENT APPLICATION**
published in accordance with Art. 158(3) EPC

(43) Date of publication:
**12.12.2007 Bulletin 2007/50**

(51) Int Cl.:
*A61K 31/7016* (2006.01)  *A61P 1/14* (2006.01)
*A61P 3/04* (2006.01)  *A61P 3/06* (2006.01)
*A61P 3/10* (2006.01)  *A61P 43/00* (2006.01)
*A23L 1/30* (2006.01)  *C07H 3/04* (2006.01)

(21) Application number: **06730209.1**

(22) Date of filing: **28.03.2006**

(86) International application number:
**PCT/JP2006/306261**

(87) International publication number:
**WO 2006/104137 (05.10.2006 Gazette 2006/40)**

(84) Designated Contracting States:
**DE FR GB**

(30) Priority: **29.03.2005 JP 2005095747**
              **29.09.2005 JP 2005284665**

(71) Applicant: **Mitsui Sugar Co., Ltd.**
**Chuo-ku**
**Tokyo 103-8423 (JP)**

(72) Inventors:
• **KASHIMURA, Jun,**
  **Research Laboratory Corporate**
  **Chigasaki-shi, Kanagawa 2530042 (JP)**

• **NAGAI, Yukie,**
  **c/o Research Laboratory Corporate**
  **Chigasaki-shi, Kanagawa 2530042 (JP)**
• **EBASHI, Tadashi,**
  **c/o Research Laboratory Corporate**
  **Chigasaki-shi, Kanagawa 2530042 (JP)**
• **GODA, Toshinao**
  **Shizuoka-shi, Shizuoka 4240857 (JP)**

(74) Representative: **Bawden, Peter Charles**
**Bawden & Associates,**
**4 The Gatehouse,**
**2 High Street**
**Harpenden,**
**Hertfordshire AL5 2TH (GB)**

(54) **SUCRASE ACTIVITY INHIBITOR, GLUCOAMYLASE ACTIVITY INHIBITOR AND FOOD AND FEED CONTAINING THE SAME**

(57) The invention provides a sucrase activity inhibitor and a glucoamylase activity inhibitor comprising one or more saccharides selected from the group consisting of palatinose, trehalulose and palatinit. Further, the invention provides a food or feed comprising the sucrose activity inhibitor and/or the glucoamylase activity inhibitor.

Fig. 1
Fig.1

EP 1 864 670 A1

**Description**

Technical Field of the Invention

The present invention relates to a sucrase activity inhibitor and a glucoamylase activity inhibitor comprising one or more saccharides selected from the group consisting of isomaltulose (or palatinose: trademark), trehalulose and isomalt (or palatinit: trademark). The present invention relates also to a food or feed comprising the sucrase activity inhibitor and optionally sucrose. Further, the present invention relates to a food or feed comprising the glucoamylase activity inhibitor and optionally starch and/or dextrin.

Background Art

Recently, a variety of saccharide-decomposition enzyme inhibitors have attracted attention for prevention of lifestyle-related diseases. In particular, the effects of sucrase- and maltase-activity inhibitors have been studied for the purposes of (1) suppressing intestinal absorption of saccharides to thereby prevent elevation of blood sugar level and prevent accumulation of fat, (2) slowing digestion of saccharides down to carry saccharides into large intestine so as to lower caloric intake, and (3) having saccharides ingested by beneficial enterobacteria to thereby control intestinal disorders.

Mammals primarily take starch as saccharinity or saccharides. Once mammals take starch, the starch is decomposed by salivary amylase and pancreatic amylase at random. Here, almost no glucose is formed, and di- to octosaccharides such as maltose, maltotriose and $\alpha$-limit dextrin are main products. These disaccharides and oligosaccharides derived from food or saccharides mentioned above are decomposed into monosaccharides by saccharide-decomposition enzymes that are localized in small intestine, such as isomaltase, lactase, maltase, sucrase, trehalase and glucoamylase, and absorbed.

Conventionally, maltase that decomposes maltose and sucrase that decomposes sucrose which is a typical sugar sweetener were typical saccharide-decomposition enzymes. Now, it is known that enzymes having a maltase activity are mostly sucrase-isomaltase complexes and these complexes demonstrate 80% of the maltase activity (see the following Non-Patent Publication 1). The sucrase-isomaltase complexes also demonstrate all of the sucrase activity and almost all (approximately 99%) of the isomaltase activity besides the maltase activity. Meanwhile, maltase-glucoamylase complexes demonstrate the remaining 20% of the maltase activity and, further, all of the intestinal neutral glucoamylase activity and 1% of the isomaltase activity.

The maltase activity refers to an activity of decomposing disaccharide maltose to produce two glucose molecules. The glucoamylase activity refers to an activity of decomposing, at terminals, a partially decomposed product of starch (i.e., amylose and amylopectin), in which glucoses are bonded primarily by $\alpha$-1,4 bond and partially by $\alpha$-1,6 bond, to produce each one molecule of glucose. It is reported that the sucrase-isomaltase complex demonstrates the maltase activity utilizing maltose as a substrate, but does not demonstrate the glucoamylase activity utilizing amylose or amylopectin as a substrates. The sucrase activity refers to an activity of recognizing a glucose site of sucrose and decomposing it into glucose and fructose. In mammals, this activity is present only in the sucrase-isomaltase complex. Invertase which is brought by microorganisms is same as sucrase in hydrolyzing sucrose into glucose and fructose. However, the invertase is $\beta$-fructofuranosidase which recognizes a fructose portion of sucrose to be decomposed and, therefore, is a different enzyme having a recognition site different from that of the sucrase activity, and demonstrates a different activity.

The following activity inhibitors against saccharide-decomposition enzymes have been reported.

D-xylose and L-arabinose are reported to have a sucrase activity inhibitory effect (see Patent Publication 1). L-fucose, 2-deoxy-D-galactose, L-xylose, D-ribose, D-tagatose, D-ribrose, D-lyxose and D-xylulose are reported to have an $\alpha$-glucosidase activity inhibitory effect (see Patent Publication 2). In relation to these, oligosaccharides composed of xylitol and xylose, xylose derivatives, saccharides such as arabitol, erythrose, erythritol and glyceraldehyde are reported to have an $\alpha$-glucosidase activity inhibitory effect (see Patent Publications 3 and 4). The afore-mentioned saccharides are primarily monosaccharides or monosaccharide alcohols. Even when these are disaccharides or oligosaccharides, they contain xylose as a constituent. These saccharides themselves used as an enzymatic activity inhibitor are rarely digested and absorbed and express only the inhibitory effect on the enzymatic activities. The $\alpha$-glucosidase in a broad sense is a general name for enzymes that recognize an $\alpha$-glucoside bond and hydrolyze it, including numerous enzymes such as sucrase, maltase, and glucoamylase. However, in a narrower sense, the $\alpha$-glucosidase means maltase and sucrase. The enzymes and enzymatic activities included in the $\alpha$-glucosidase provide each different substrate specificities as described above.

For saccharide-digestive enzyme inhibitors derived from plants, it is reported that a plant extract of *Juglandaceae Juglans sp.* inhibits $\alpha$-glucosidase and amylase activities (see Patent Publication 5); a hot water extract from a banaba, *Lagerstroemia speciosa (Linn) Pers,* inhibits maltase, sucrase, glucoamylase and isomaltase activities (see Patent Publication 6); and caffeoyl isolated and purified from yacon stems and leaves or coffee beans inhibits maltase activity (see Patent Publication 7).

It is also reported that a combination of sucrose and a sucrase inhibitor can be used as a proliferation promoter for intestinal bifidobacterium, and the aforementioned saccharides such as L-arabinose, D-xylose, D-ribose and D-tagatose are mentioned as the sucrase inhibitor to be used (see Patent Publication 8).

As other saccharide-decomposition enzyme inhibitors, a novel amino sugar produced by *genus Streptomyces* (*actinomycetes*) is disclosed to inhibit the amylase activity (see Patent Publications 9 and 10).

It has been reported that palatinose and trehalulose are, like isomaltose, decomposed by isomaltase and do not inhibit the sucrase activity (see Non-Patent Publications 2 and 3). In tests to confirm any inhibitory effect of palatinose on the sucrase activity, using 4.3 - 56 mM (approximately 0.15-1.9 wt. %) of sucrose as a substrate and 14 mM (approximately 0.48 wt. %) of palatinose as an inhibitor, it is reported that palatinose has no inhibitory effect. In tests to confirm any inhibitory effect of trehalulose on the sucrase activity, using 5-75 mM (approximately 0.17-2.6 wt. %) of sucrose as a substrate and 5 mM (approximately 0.17 wt. %) of trehalulose as an inhibitor, it is reported that trehalulose has no inhibitory effect. On the other hand, palatinose and palatinit are reported to have a maltase activity inhibitory effect to inhibit a reaction using maltose as a substrate (see Non-Patent Publication 4). However, because the enzyme used was not purified in the Non-Patent Publication 4, it is unknown whether that was inhibition of the sucrase activity or inhibition of the activity of the maltase-glucoamylase complex. Also, inhibition of decomposition of dextrin and starch are not reported there.

Patent Publication 1: WO 94/12057
Patent Publication 2: Japanese Patent Application Laid-open No. Hei 6-065080
Patent Publication 3: Japanese Patent Application Laid-open No. Hei 8-023973
Patent Publication 4: Japanese Patent Application Laid-open No. Hei 11-286449
Patent Publication 5: Japanese Patent Application Laid-open No. 2004-352649
Patent Publication 6: Japanese Patent Application Laid-open No. 2002-012547
Patent Publication 7: Japanese Patent Application Laid-open No. 2002-255806
Patent Publication 8: Japanese Patent Application Laid-open No. 2004-113068
Patent Publication 9: Japanese Patent Application Laid-open No. Sho 56-125398
Patent Publication 10: Japanese Patent Application Laid-open No. Sho 54-092909
Non-Patent Publication 1: B. L. Nichols, S. Avery, P. Sen, D. M. Swallow, D. Hahn and E. Sterchi, Proceedings of the National Academy of Sciences USA, Vol. 100 (3), 1432~1437, 2003.
Non-Patent Publication 2: T. Goda and N. Hosoya, Nippon Eiyo Shokuryo Gakkaishi (in Japanese) (Journal of Japanese Society of Nutrition and Food Science), Vol. 36 (3), 169-173, 1983.
Non-Patent Publication 3: K. Yamada, H. Shinohara, and N. Hosoya, Nutrition Reports International, Vol. 32 (5), 1211-1220, 1985.
Non-Patent Publication 4: S.C. Ziesenitz, Zeitschrift fur Ernahrungswissenshaft, Vol. 25, 253-258, 1986.

Summary of the Invention

Problems to Be Solved by the Invention

The prior art saccharide-decomposition enzyme inhibitors act only as an inhibitor and are difficult to be digested and absorbed. Accordingly, these are not intended to serve as a nutrition source. Therefore, an amount of these added to exhibit the effect must be very small relative to an amount of saccharide that is a target substrate of the inhibition of decomposition, such as starch, maltose and sucrose. When an inhibitor is incorporated together with a substrate saccharide into a food or food ingredient, it is a delicate job to set an appropriate amount to be taken. If an inhibitor and saccharide are taken in an excessive amount, a larger quantity of saccharide reach large intestine in an undigested state to cause a problem of developing digestive disorders such as laxative property and sensation of fullness.

Plant extracts among the conventional saccharide-decomposition enzyme inhibitors are generally brownish. Therefore, only a limited amount of the plant extract can be added to a food due to the color.

Moreover, many of the conventional saccharide-decomposition enzyme inhibitors have unique tastes such as bitter taste and astringency or odor. For this reason, it is not pleasant to have them as such, and, further, only a limited amount of them can be used in a food due to the taste added to the food and change in odor.

Sucrase inhibition and maltase inhibition have been investigated as a primary object for effects of the saccharide-decomposition enzyme inhibitors to prevent lifestyle-related diseases and obesity and to control intestinal disorders. We have carefully read various reports to find that the terms, sucrase and maltase, are not the names of such enzymes, but mean enzymes which have a sucrose decomposition activity (sucrase activity) and a maltose decomposition activity (maltase activity), respectively. It was known that the aforesaid sucrase-isomaltase complex account for all of the sucrase activities and most of the maltase activity in mammals. Thus, the sucrase activity inhibitory effect and the maltose activity inhibitory effect both correspond mainly to the inhibitory effects on the activity of the sucrase-isomaltase complex. Because this complex does not have a decomposition activity for amylose and amylopectin which are constituents of starch and dextrin, substantially no inhibitory effects to decomposition of starch and dextrin were confirmed.

A report that palatinose and trehalulose do not inhibit the sucrase activity was based on the results of the tests which were conducted at a low concentration of s substrate sucrose of 2.6% or lower and at a low concentrations of the inhibitor palatinose and trehalulose of less than 0.5 wt. %. These concentrations are so high as 10% or greater proportions relative to the substrate concentrations, but are too low for the expression of saccharide-decomposition enzyme inhibitory effects. In order to express the saccharide-decomposition enzyme inhibitory effects, the concentrations must be enough to inhibit the enzymes present in small intestine. Therefore, they should have been taken at a higher concentration enough as an inhibitor concentration

Means to Solve the Problems

The inventors have found that one or more saccharides selected from the group consisting of isomaltulose (or palatinose: trademark), trehalulose and isomalt (or palatinit: trademark) inhibit a sucrase activity and a glucoamylase activity to complete the present invention.

Palatinose, trehalulose and palatinit have been commonly used as a sugar sweetener in drinks and foods. Palatinose and trehalulose are structural isomers of sucrose and are disaccharides composed of glucose and fructose. Palatinose and trehalulose are completely decomposed by isomaltase into glucose and fructose. These monosaccharides are absorbed in small intestine and metabolized, like sucrose is decomposed enzymatically. Accordingly, these disaccharides are safe nutritional sugars. Therefore, when palatinose and trehalulose are taken together with sucrose or starch or dextrin, or any combinations thereof, they are digested and absorbed in small intestine, without causing any gastrointestinal disorders such as laxative actions or sensation of abdominal fullness. That is, when palatinose and trehalulose are used as the inhibitor, there is no particular restriction on the amount to be taken.

Palatinit is a type of sugar alcohol and is a hardly digestible saccharide like sorbitol and maltitol. A maximum no-effect level of palatinit has been determined as 0.3g/kg bodyweight, so that it can be used without any problem in an amount less than this level.

Effects of the Invention

According to the present invention, the sucrase activity can be inhibited by taking a sucrase activity inhibitor comprising at least one selected from palatinose, trehalulose and palatinit as an active ingredient, together with sucrose intake or before or after sucrose intake. Also, the glucoamylase activity can be inhibited by taking a glucoamylase inhibitor comprising at least one selected from palatinose, trehalulose and palatinit as an active ingredient, together with starch or dextrin intake or before or after starch or dextrin intake.

Palatinose, trehalulose and palatinit are sugar sweeteners themselves and are food items. Palatinose and palatinit are white powder, and trehalulose is colorless liquid like syrup. Further, palatinose and palatinit present a sweet taste which is close to that of sucrose and do not have any undesirable tastes such as bitter taste, astringency or sourness. Therefore, no color or undesirable taste is added to a food, when palatinose, trehalulose and palatinit are used in the food.

The sucrase inhibitor and the glucoamylase inhibitor of this invention containing palatinose or trehalulose as an active ingredient can be used without any particular limitation on the amount of intake of palatinose and trehalulose. When palatinit is used, the inhibitor can be used at below the ordinary maximum no-effect level of palatinit. Therefore, when at least one selected from the group consisting of palatinose, trehalulose and palatinit is used, the amount of intake of the inhibitor is not particularly limited. When the inhibitor is used in combination with sucrose or starch or both in foods, the amount of intake is not limited.

Best Modes for Working the Invention

Preferred embodiments of the present invention will be described below in detail.

"Palatinose" (trademark) in the present specification refers to a disaccharide in which glucose is bound to fructose via $\alpha$-1,6-glucosyl bond, and is isomaltulose.

"Trehalulose" in the present specification refers to a disaccharide in which glucose is bound to fructose via $\alpha$-1,1-glucosyl bond.

"Palatinit" (trademark) in the present specification refers to sugar alcohol that is obtained by hydrogenation of palatinose, and is a mixture of $\alpha$-D-glucopyranosyl-1,6-D-sorbitol (abbreviated as GPS) and its isomer, $\alpha$-D-glucopyran-

osyl-1,1-D-mannitol (abbreviated as GPM) with two molecules of crystalline water being attached to one molecule of GPM. Palatinit is isomalt. The maximum no-effect level of palatinit for Japanese people is 0.3g/kg bodyweight.

"Sucrase activity" in the present specification refers to an activity of recognizing a glucose site of sucrose and decomposing sucrose into glucose and fructose. "Sucrase activity inhibition" refers to partial or complete inhibition of the sucrase activity.

"Glucoamylase activity" in the present specification refers to an activity of decomposing, at terminals, a partial decomposition product of starch in which glucose is bound primarily via an $\alpha$-1,4-bond (amylose and amylopectin) to produce each one glucose per decomposition. "Glucoamylase activity inhibition" refers to partial or complete inhibition of the glucoamylase activity. A partial decomposition product of starch is generally called dextrin, but the decomposition activity for maltooligosaccharide (4 or more saccharides) which has a smaller molecular weight than dextrin is also called glucoamylase activity.

An enzyme having the sucrase activity is different from an enzyme having the glucoamylase activity. The enzyme having the sucrase activity is a sucrase-isomaltase complex which is localized in small intestine. The enzyme having the glucoamylase activity is glucoamylase alone in small intestine. It has been confirmed in the following confirmation tests that starch or dextrin was decomposed by glucoamylase in the following Examples and the sucrase-isomaltase complex had no or little dextrin decomposition activity.

As the enzyme activity inhibitor comprising palatinose as an active ingredient in the present invention, use may be made of, for example, crystalline palatinose, palatinose syrup or trehalulose syrup. Crystalline palatinose (trade name: Crystalline Palatinose IC, ex Mitsui Sugar Co., Ltd.) contains 99.0% or more of palatinose including crystalline water. Palatinose syrup (trade name: Palatinose Syrup-ISN or -TN, ex Mitsui Sugar Co., Ltd.) contains 11 to 17% of palatinose and 53 to 59% of trehalulose. Trehalulose syrup (trade name: Mildear-75 or - 85, ex Mitsui Sugar Co., Ltd.) contains 8 to 13% of palatinose and 83 to 89% of trehalulose.

As the enzyme activity inhibitor comprising trehalulose as an active ingredient in the present invention, use may be made of, for example, palatinose syrup or trehalulose syrup. Examples of palatinose syrup and trehalulose syrup are as described above.

As the enzyme activity inhibitor comprising palatinit as an active ingredient in the present invention, use may be made of, for example, palatinit (trade name: Palatinit PN series and GS series, ex Mitsui Sugar Co. Ltd.). Palatinit PN series contain $50\pm5\%$ of GPM and $50\pm5\%$ of GPS. Palatinit PN, Palatinit PNS-2, Palatinit PNM-2, Palatinit PNP, etc. have different particle sizes. In addition, Palatinit GS series contain $20\pm5\%$ of GPM and $80\pm5\%$ of GPS. Palatinit GS in a granular state and Palatinit GSP in a powder state are also available.

The sucrase activity inhibitor and the glucoamylase activity inhibitor of the present invention (hereinafter, referred to as the present inhibitors) may have any form. Besides the aforementioned ones, fondants, granules, tablets, syrups, drinks or powdery mixtures comprising palatinose, trehalulose or palatinit may be included. In the following Examples 12 and 13, the inhibitors of the present invention in a granular form containing palatinose and palatinit are described. In addition, the present inhibitors may be combined with materials that can be used in foods, quasi drugs, medicines or the like, and can be processed into functional foods, health foods, quasi drugs, medicines or the like.

In the following Examples 14 through 30, described are the inhibitors or foods comprising at least one of palatinose, trehalulose and palatinit. When these inhibitors or foods are taken together with saccharides such as sucrose, dextrin or starch that are a substrate for one or both of sucrase and glucoamylase, the inhibitors or foods inhibit decomposition of substrate saccharides in small intestine on account of the sucrase activity inhibitory effect or the glucoamylase activity inhibitory effect of palatinose, trehalulose or palatinit.

In Examples 31 through 37, described are foods comprising at least one of palatinose, trehalulose and palatinit. Examples 38 through 40 demonstrate feeds comprising at least one of palatinose, trehalulose and palatinit. These foods and feeds also contain saccharides such as sucrose, dextrin and starch that are a substrate for one or both of sucrose or glucoamylase. These foods and feeds inhibit decomposition of the substrate contained in the foods and feeds in small intestine on account of palatinose, trehalulose or palatinit contained in the foods and feeds.

If sucrose which is a substrate for sucrase or a partial decomposition product of starch and/or dextrin which are a substrate for glucoamylase are present in small intestine when one or more saccharides selected from the group consisting of palatinose, trehalulose and palatinit reaches small intestine, decomposition of the sucrose or the partial decomposition product of starch and/or dextrin is inhibited, so that total digestive absorption of these substrates and the enzymatic activity inhibitors is suppressed, compared to a case where no enzymatic activity inhibitor is present.

The amount of intake of the present inhibitors is not particularly restricted. In a case where palatinose, trehalulose and/or palatinit are taken in combination with sucrose and/or starch and/or dextrin, the inhibitory effect on the sucrase activity and the glucoamylase activity can be expected under such conditions that where a concentration of sucrose, when taken orally as usual, is 5 to 50 wt. % or a concentration of starch and/or dextrin is 5 to 70 wt. %, a palatinose concentration is 0.5 wt. % or larger and a ratio of palatinose to a total of sucrose or starch and/or dextrin plus palatinose ranges from 0.10 to 0.90. [0038] An emptying rate from a stomach to small intestine of saccharides composed of glucose or fructose as a structural sugar, such as glucose, sucrose, maltose, dextrin, starch and fructose, is limited. If a large amount of these saccharides or a high concentration of these saccharides is taken, they are diluted in the stomach and, at the same time, the emptying rate from the stomach to the small intestine is controlled so that a concentration of these saccharides in the small intestine is suppressed below approximately 10%. ("Satou-hyakka" (in Japanese) by Akikazu Takeda, Hitoshi Hashimoto and Hiroshi Ito, pp 11 to 12, published by the Sugar Industry Association inc. and the Japan Sugar Refiners' Association). In the present Examples, the conditions for determining the present effect are similar with those in the small intestine. In a case where a substrate sucrose concentration was 15% or less or a substrate dextrin concentration was 10% or less, the effects of the present invention was confirmed with 10 wt. % or more, relative to the substrate, of at least one of the inhibitor selected from palatinose, trehalulose and palatinit. It is less likely that the substrate is transferred to small intestine at a higher concentration. Therefore, even when a certain high concentration of sucrose and/or starch and/or dextrin as mentioned above is taken, the present inhibitor works effectively.

In one aspect of the present invention, 10 parts by weight or more of the present inhibitor can be taken per 100 parts by weight of sucrose/or starch and/or dextrin taken or to be taken, prior to or together with having a meal; or after having a meal, or before the sucrose, starch or dextrin is decomposed in the small intestine (in a period where these are mixed in the stomach and the small intestine). Alternatively, a food or feed can contain the present inhibitor together with sucrose, starch or dextrin.

In another aspect of the present invention, a food or feed comprising the present sucrase activity inhibitor contains 3 wt. % or more of sucrose and 10 wt. % or more, relative to the weight of the sucrose, of the sucrase activity inhibitor (see the following Examples 8 and 9). In a case where the sucrase activity inhibitor is palatinit, the sucrase inhibitory activity is appreciable with 0.5 wt. % or more of sucrose and 10 wt. % or more, relative to the weight of sucrose, of palatinit (see Example 9).

A food or feed comprising the present glucoamylase activity inhibitor contains starch and/or dextrin in an amount of 2 wt. % or more and the glucoamylase activity inhibitor in an amount of 2 wt. % or more relative to the weight of the starch and/or dextrin (see Examples 10 and 11). In a case where the glucoamylase inhibitor is palatinit, the glucoamylase inhibitory activity is appreciable with 0.5 wt. % or more of starch and/or dextrin and 10 wt. % or more, relative to the weight of starch and/or dextrin, of palatinit (see Example 11 below).

The present inhibitor can be used also as drugs for controlling intestinal disorders, sustainable energy feeders, drugs for sustaining perception of satiety, drugs for preventing fat accumulations and obesity, and stabilizers for a blood glucose level.

Other embodiments of the present invention are a method of controlling intestinal disorders, a method of supplying sustainable energy, a method of sustaining perception of satiety, and a method of suppressing fat accumulation, a method of preventing obesity, and a method of stabilizing a blood glucose level, using one or more saccharides selected from the group consisting of palatinose, trehalulose and palatinit.

The food comprising the present inhibitors may be in any form, such as drinks comprising carbohydrates (sports drinks, jelly drinks, carbonated drinks), processed foods, confectionaries (candies), breads, and pancakes and so on.

The feed comprising the present inhibitors may be in any form, such as solid feeds and liquid feeds. When the present inhibitor is given to commercial animals (pigs, cattle and domestic fowl) and companion animals (cats and dogs), the effects of controlling intestinal disorders and stabilization of a blood glucose level are expected to be attained.

A package containing the food or feed comprising the present inhibitor may be accompanied with an instruction describing the inhibitory effect.

Other embodiments of the present invention are a method of using one or more saccharides selected from the group consisting of palatinose, trehalulose and palatinit for producing a drug for suppressing fat accumulation, a drug for preventing obesity, a drug for stabilizing a blood glucose level and a drug for controlling intestinal disorders.

Examples

The present invention will be explained with reference to the following Examples. However, this invention shall not be limited by these Examples. Unless otherwise specified, "%" in the Examples means % by w/v.

[Confirmation Tests]

Decomposability of the sucrase-isomaltase complex on dextrin was investigated in order to confirm that decomposition of starch in the following Examples 4, 6 and 7 and Test Example 5 was attained by glucoamylase, but not by sucrase or isomaltase.

1. Test Methods

A. Extraction of a crude enzyme, sucrase-isomaltase complex

Twenty grams of rat small-intestinal acetone powder (ex Sigma Corporation) were added to 180 ml of 50 mM potassium phosphate buffer (pH 7.0) and extracted for 4 hours while slowly stirred at room temperature, to which 6.5 ml of a cysteine solution (100 mg/10 ml) and 1.5 ml of a papain solution (MP Biomedicals, LLC 80 mg/2 ml) were subsequently added and the mixture was incubated at 37°C for 1 hour to conduct a papain treatment. Then, a supernatant was recovered by centrifugal separation (8000 rpm x 15 min.) and further filtered by aspiration using Toyo Filter Paper No. 2 to obtain 164 ml of a crude enzyme solution. Protein was precipitated at an ammonium sulfate concentration of 45% from the crude enzyme solution and then a supernatant was recovered by centrifugal separation (8000 rpm x 15 min.). Next, ammonium sulfate was added to the supernatant up to a concentration of 65% to precipitate protein, and then the precipitate was recovered by centrifugal separation (8000 rpm x 15 min.). The precipitate was dissolved in a 10 mM potassium phosphate buffer (pH 7.0) and the solution was placed in a dialysis tube (dialysis membrane, ex the Union Carbide Corporation). The dialysis tube was dipped in 2 liters of 10 mM potassium phosphate buffer (pH 7.0) and left overnight for dialysis and desalting while slowly stirred, resulting in 50 ml recovered. Next, the desalted one was separated by column chromatography with DEAE-Cellulose (50 ml volume) and fractions (each 12 ml/tube) were collected using a fraction collector. The chromatographic separation was carried out while elevating a salt concentration of the potassium phosphate buffer (pH 7.0) stepwise (10 mM→50 mM →100 mM). The enzymatic activity was measured for each fraction and the fraction with the highest activity of a sucrase-isomaltase complex was recovered to obtain a 12 ml crude enzyme solution. The sucrase-isomaltase complex activity was determined by measuring a decomposition activity for sucrose in each fraction. More specifically, a 28 mM sucrose solution and 0.125 ml of a fraction were placed in a 2.5 ml small test tube, shaken at 37°C for 20 min. at 60 strokes/min., then the small test tube was dipped in boiling water for 3 min. to inactivate the enzyme. A glucose concentration was determined using the F-kit Glucose (ex Roche) described in item D below.

B. Sample Preparation

Each three samples for the following three kinds of samples were prepared in small test tubes so as to result in a final volume of 2.5 ml.

Sample 1 Dextrin 2% (W/V)/crude enzyme liquid 0.125 ml
Sample 2 Dextrin 2% (W/V)/crude enzyme liquid 0.25 ml
Sample 3 Sucrose 26.6 mM (0.91 % (W/V)/crude enzyme liquid 0.125 ml

Dextrin used was dextrin from corn (Type 3) (ex Sigma Corporation, USA). Sucrose was a guaranteed reagent Saccharose (ex Wako Pure Chemical Industries Ltd.). Each concentration means a final concentration of the dextrin or sucrose.

C. Reaction Conditions

Each sample was shaken at 37°C for 20 min. at 60 strokes/min., and the small test tube was dipped in boiling water for 3 min. to inactivate the enzyme.

D. Measurement of a Glucose Concentration

A glucose concentration in each sample after the reaction was measured using the F-kit Glucose (ex Roche Corporation). A liberated glucose concentration (g/l) was calculated by subtracting a blank value (0.1 M phosphate buffer alone (pH 6.8)) and a concentration of glucose originally contained in the test sample (dextrin and sucrose) from the determined glucose concentration.

E. Confirmation of a Disaccharide Content in Dextrin

Dextrin is a polymer of glucose. Generally, product dextrin contains monosaccharide (glucose), disaccharides (maltose and isomaltose) and oligosaccharides such as tri- or higher saccharides. The amount of glucose (monosaccharide) that was originally present in the product dextrin can be measured by the F-kit Glucose as mentioned above, and is subtracted in the calculation. However, maltose that is a substrate for sucrase and isomaltose that is a substrate for isomaltase were not subtracted. If maltose or isomaltose is present in the reaction solution, they are decomposed by the sucrase-isomaltase complex to liberate glucose. Therefore, a content of disaccharides in the product dextrin was determined by liquid chromatography. The measurement conditions were as follows.
Column: Sugar KS-801 and Sugar KS-802 (ex Showa Denko K. K.) were connected with each other.
Mobile phase: water
Flow rate: 1 ml/min.
Column temperature: 60°C.
It was found that the product dextrin used contained 1.3 wt. % of disaccharides, based on a solid content of the dextrin.

2. Results

The mean amounts (g/l) of produced glucose in Samples 1 to 3 were 0.057, 0.088, and 0.474, respectively. Although the dextrin concentration of Sample 1 (2 wt. %) was higher than the sucrose concentration of Sample 3 (0.91 wt. %), the amount of produced glucose in Sample 1 was at a level of 1/10, compared to the amount of produced glucose in Sample 3. Although the sucrase-isomaltase complex (crude enzyme) was not completely purified, the amount of glucoamylase was very small. Accordingly, it is understood that maltose and isomaltose that are present in the product dextrin are easily decomposed, compared to the polymeric dextrin. The product dextrin used contained 1.3 wt. % of disaccharides. It seems that the glucose produced was derived from these disaccharides. Therefore, the sucrase-isomaltase complex (crude enzyme) seems to contain almost no glucoamylase. It was confirmed by this confirmation test that the enzyme presenting the sucrase activity and the enzyme presenting the dextrin or starch decomposition activity in the rat small intestine powder used in the Examples are different from each other as described in Non-Patent Publication 1.

Example 1

(Confirmation of the maltase activity inhibitory effect and the sucrase activity inhibitory effect by palatinose)

Preparation of a Test Solution:

Palatinose (trade name: Palatinose IC, ex Shin Mitsui Sugar Co., Ltd.) (test sample) was used as a sample for investigating each enzyme activity inhibitory effect. Maltose and sucrose were used as substrates for maltase and sucrase, respectively. The test sample and the substrates were dissolved in 0.1 M phosphate buffer (pH 6.8) at the concentration shown in Table 1 below to prepare test solutions (Solutions 4 and 5). Also, the test sample or the substrates were dissolved in 0.1 M phosphate buffer (pH 6.8), respectively to at the concentration shown in Table 1 below to prepare control solutions (Solution 1 for the test sample and Solutions 2 and 3 for the substrates).

Preparation of a Small-Intestinal Enzyme Solution:

Two grams of rat small-intestinal acetone powder (ex Sigma Corporation) were dissolved in 20 ml of 0.1 M phosphate buffer (pH 6.8), left standing one day at 5°C, and subsequently subjected to centrifugal separation (8000 rpm x 15 min.). The supernatant was filtered through a 0.8 $\mu$m membrane filter to obtain a small-intestinal enzyme liquid. This liquid contained a mixture of enzymes present in small intestine, such as sucrase, glucoamylase, maltase, and isomaltase.

Method for Determining an Activity:

Each 0.25 ml of the small-intestinal enzyme liquid was added to each 5 ml of Solutions 1 through 5 and placed in a water bath at 37°C to allow them to react for 60 min. while shaken at 60 strokes/min. After the reaction, they were heated in a boiling water bath for 3 min. for inactivation of the enzymes.
The glucose concentration in each solution after the reaction was measured using the F-kit Glucose (ex Roche). A liberated glucose concentration (g/l) was calculated by subtracting the blank value (0.1 M phosphate buffer alone) and the concentration of glucose originally contained in the test sample from each glucose concentration measured. The results are as shown in Table 1.
Next, each of the liberated glucose concentration was input in the following equation to obtain decomposition inhibition ratio for maltase and sucrase. In the present tests, the substrates and the inhibitors were decomposed by enzymes present in the small-intestinal enzyme liquid. However, the enzymes decomposing the substrates are different from the enzymes decomposing the inhibitors. The decomposition inhibition ratio was determined as a measure for the inhibitory effect. When the inhibitor was added, the glucose concentration was lower than that seen when the substrate alone was reacted. A higher decomposition inhibition ratio means a stronger inhibitory effect. The results are as shown in Table 1.

[Equation 1]

$$\text{Decomposition inhibition ratio, \% = \{(A+B) - (AB)\} \div (A+B) \times 100}$$

wherein (A+B) indicates a sum of a liberated glucose concentration in the control solution containing the test sample alone after the reaction (A) and a liberated glucose concentration in the control solution containing the substrate alone after the reaction (B); and (AB) indicates a liberated glucose concentration in the test solution containing the test sample (A) and the substrate (B), after the reaction.

[Table 1]

Table 1

| Sample | Liberated glucose concentration(g/l) | Decomposition inhibition ratio, %, against maltase | Decomposition inhibition ratio, %, against sucrase |
|---|---|---|---|
| Sotution-1 5% palatinose | 0.095±0.010 | | |
| Solution-2 5% maltose | 4.956±0.423 | | |
| Solution-3 5% sucrose | 0.959±0.022 | | |
| Solution-4 5% maltose + 5% palatinose | 4.757±0.342 | 6 | |
| Solution-5 5% sucrose + 5% palatinose | 0.803±0.055 | | 24 |

As seen in Table 1, palatinose demonstrated decomposition inhibition against maltase and decomposition inhibition against sucrase. Thus, palatinose has a maltase activity inhibitory effect and a sucrase activity inhibitory effect. It has now been found that the palatinose has the sucrase activity inhibitory effect, though it is known in literature that palatinose has the maltase activity inhibitory effect.

[Test Example 1]

The procedures in Example 1 were repeated with the exception that two kinds of indigestible dextrin (trade names: Fibersol 2 and Pine Fiber Bi, ex Matsutani Chemical Industry Co., Ltd.) were used as test samples. The test samples and the substrates were dissolved in 0.1 M phosphate buffer (pH 6.8) to attain the concentration shown in Table 2 to thereby prepare test solutions (Solutions-8 through -11). Also, the test samples or substrates were dissolved in 0.1 M phosphate buffer (pH 6.8) to attain the concentration shown in Table 2 to thereby prepare control solutions (Test Sample Solutions-6 and-7 and Substrate Solutions-2 and-3).

Each 5 ml of the aforementioned test solutions and control solutions was subjected to the enzymatic reaction in the same procedures as in Example 1. The liberated glucose concentration, the decomposition inhibition ratio for maltase and the decomposition inhibition ratio for sucrase were calculated for each solution after the reaction. The results are as shown in Table 2.

[Table 2]

Table 2

| Sample | Liberated glucose concentration (g/l) | Decomposition inhibition ratio,%, against maltase | Decomposition inhibition ratio, %, against sucrase |
|---|---|---|---|
| Solution-6 5% Fibersol 2 | 1.176±0.093 | | |
| Solution-7 5% Pine Fiber Bi | 7.711±0.130 | | |
| Solution-2 5% maltose | 4.956±0.423 | | |
| Solution-3 5% sucrose | 0.959±0.022 | | |

(continued)

| Sample | Liberated glucose concentration (g/l) | Decomposition inhibition ratio,%, against maltase | Decomposition inhibition ratio, %, against sucrase |
|---|---|---|---|
| Solution-8 5% maltose+5% Fibersol 2 | 4.829±0.359 | 21 | |
| Solution-9 5% maltose+5% Pine Fiber Bi | 10.563±0.929 | 17 | |
| Solution-10 5% sucrose+5% Fibersol 2 | 1.683±0.103 | | 21 |
| Solution-11 5% sucrose+5% Pine Fiber Bi | 8.275±0.385 | | 5 |

As seen in Table 2, the decomposition rates of Fibersol 2 and Pine Fiber Bi were much higher than the decomposition rate of sucrose. This is probably because Fibersol 2 and Pine Fiber Bi which are indigestible dextrin contain 5 to 15% and 45 to 55% of digestible components (components other than dietary fiber), respectively, and the decomposition rates of the latter were comparable to that of maltose or dextrin.

To compare Solution-4, Solution-8, and Solution-9 in Tables 1 and 2 where palatinose, Fibersol 2, or Pine Fiber Bi was added to the substrate maltose, the liberated glucose concentration in Solution-4 with palatinose was lowest, and was lower than the liberated glucose concentration of the control Solution-2 where only the substrate maltose was added. Thus, the effect of palatinose was substantially highest. Regarding the maltase activity inhibitory effect, it is seen that palatinose demonstrated the maltase activity inhibitory effect, but both indigestible dextrins demonstrated higher maltase activity inhibition ratio than that of palatinose.

As seen, to compare Solution-5, Solution-10, and Solution-11 in Tables 1 and 2 where palatinose, Fibersol 2, or Pine Fiber Bi was added to the substrate sucrose, the liberated glucose concentration in Solution-5 with palatinose was lowest, and was lower than the liberated glucose concentration in Solution-3) where only the substrate sucrose was added. Regarding the sucrase activity inhibitory effect, it is seen that palatinose demonstrated the highest effect among the test samples shown in Tables 1 and 2.

Example 2

(Confirmation of intensity of the sucrase activity inhibition by palatinose)

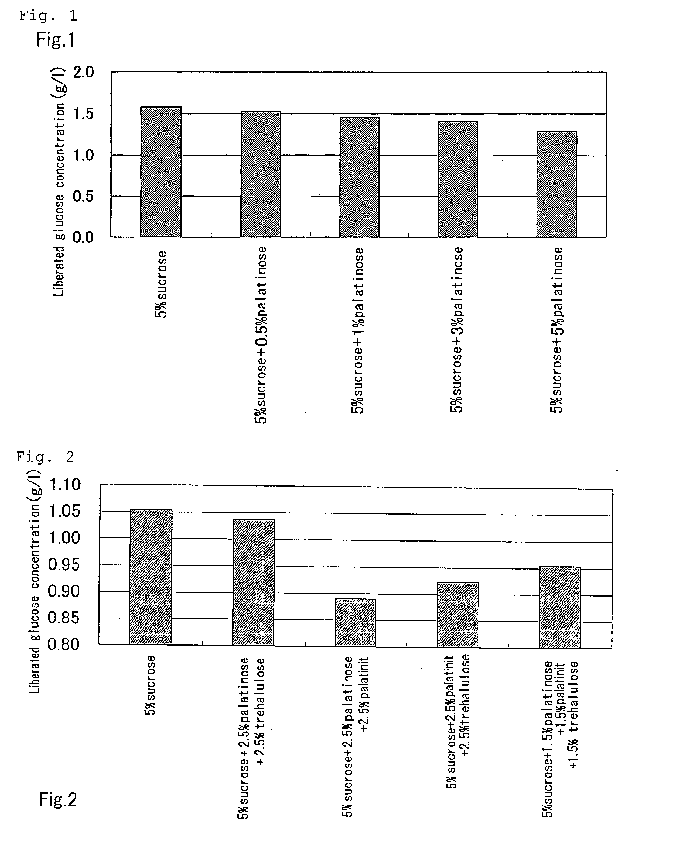

The procedures in Example 1 were repeated with the exception that the test solutions contained 5% of sucrose and 0.5%, 1.0%, 3.0% or 5.0% of palatinose to examine intensity of the sucrase activity inhibition. A control solution contained 5% of sucrose in 0.1 M phosphate buffer (pH 6.8). The results are as shown in Fig. 1.

As seen in Fig. 1, the amount of liberated glucose decreased with the increasing concentration of palatinose added, though the inhibitor palatinose itself was decomposed by the enzyme isomaltase in the intestinal enzyme solution. Therefore, it is clear that palatinose inhibits sucrase activity depending upon its concentration.

Example 3

(Confirmation of the sucrase activity inhibitory effect of palatinit, palatinose and isomaltose)

The procedures in Example 1 were repeated with the exception that palatinit and palatinose were used as test samples and sucrose was used as a substrate. The test samples and the substrate were dissolved in 0.1 M phosphate buffer (pH 6.8) to attain the concentration shown in Table 3 to thereby prepare two test solutions. Besides, each one of the test samples or the substrate was dissolved in 0.1 M phosphate buffer (pH 6.8) to attain the concentration shown in Table 3 to thereby prepare control solutions. Each 5 ml of the aforementioned test solutions or the control solutions was subjected to the enzymatic reaction according to the same procedures as in Example 1. The glucose concentration and the fructose concentration were measured. The fructose concentration was measured using the F-kit Fructose (ex Roche). The liberated fructose concentration, g/l, was obtained by subtracting the blank value (0.1 M phosphate buffer alone) from the resultant fructose concentration. The results are as shown in Table 3.

[Table 3]

Table 3

| Sample | Liberated glucose concentration (g/l) | Liberated fructose concentration (g/l) | Decomposition inhibition ratio, %, against sucrase |
|---|---|---|---|
| 5% palatinit | 0.05 | 0.00 | |
| 5% palatinose | 0.11 | 0.21 | |
| 5% sucrose | 1.01 | 1.09 | |
| 5% sucrose+5% palatinit | 0.72 | 0.81 | 31 |
| 5% sucrose+5% palatinose | 0.79 | 1.05 | 29 |

Where palatinit or palatinose was added to sucrose, both the liberated glucose concentration and the liberated fructose concentration were lower than the liberated glucose concentration and the liberated fructose concentration in the control solution with sucrose alone. This means that palatinit and palatinose have a sucrase inhibitory effect.

[Test Example 2]

(Confirmation of the sucrase activity inhibitory effect of isomaltose)

The procedures in Example 1 were repeated with the exception that isomaltose was used as a test sample. Isomaltose is a disaccharide where two molecules of D-glucose are bound with each other via $\alpha$-1,6 glucoside bond. The test sample and the substrate were dissolved in 0.1 M phosphate buffer (pH 6.8) to attain the concentration as shown in Table 4 to thereby prepare a test solution. Besides, the test sample or the substrate was dissolved in 0.1 M phosphate buffer (pH 6.8) to attain the concentration shown in Table 4 to thereby prepare control solutions. Each 5 ml of the aforementioned test solution and the control solutions was subjected to the enzymatic reaction according to the same procedures as in Example 1. The liberated glucose concentration, the liberated fructose concentration and the decomposition inhibition ratio against sucrase were determined. The results are as shown in Table 4.

[Table 4]

Table 4

| Sample | Liberated glucose concentration (g/l) | Liberated fructose concentration (g/l) | Decomposition inhibition ratio, %, against sucrase |
|---|---|---|---|
| 5% isomaltose | 2.14 | 0.02 | |
| 5% sucrose | 1.01 | 1.09 | |
| 5% sucrose + 5% isomaltose | 2.18 | 0.70 | 31 |

In the test solution where isomaltose was added to sucrose, an increased liberated glucose concentration (2.18 g/l) was detected due to decomposition of isomaltose. However, the liberated fructose concentration in this test solution, (0.70 g/l) was lower than the liberated fructose concentration in the control solution with sucrose only (1.09 g/l). Thus, isomaltose has a sucrase activity inhibitory effect. It is noted that the rate of isomaltose decomposed by isomaltase was faster than the rate of decomposition of sucrose by sucrase, so that the total concentration of liberated monosaccharides (2.18 + 0.70 = 2.88, g/l) was slightly higher than the total concentration in the control solution with sucrose only (1.01 + 1.09 = 2.10, g/l).

[Test Example 3]

(Confirmation of a lactase activity inhibitory effect by palatinit, palatinose and isomaltose)

The procedures in Example 1 were repeated with the exception that palatinit, palatinose or isomaltose was used as a test sample and lactose was used as a substrate. The test samples and the substrate were dissolved in 0.1 M phosphate buffer (pH 6.8) to attain the concentration shown in Table 5 to thereby prepare three test solutions. Besides, each of the test samples or the substrate was dissolved in 0.1 M phosphate buffer (pH 6.8) to attain the concentration shown in Table 5 to thereby prepare control solutions. Each 5 ml of the aforementioned test solutions and the control solutions was subjected to the enzymatic reaction according to the same procedures as in Example 1. The glucose concentration, the fructose concentration and the galactose concentration were measured. The galactose concentration was measured using F-kit Galactose (ex Roche). The liberated galactose concentration, g/l, was calculated by subtracting the blank value (0.1 M phosphate buffer alone) from the measured galactose concentration. The results are as shown in Table 5 below.

[Table 5]

Table 5

| Sample | Liberated glucose concentration (g/l) | Liberated fructose concentration (g/l) | Liberated galactose concentration (g/l) | Decomposition inhibition ratio, %, against lactase |
|---|---|---|---|---|
| 5% palatinit | 0.05 | 0.00 | 0.00 | |
| 5% palatinose | 0.11 | 0.21 | 0.00 | |
| 5% isomaltose | 2.14 | 0.02 | 0.00 | |
| 5% lactose | 0.23 | 0.00 | 0.16 | |
| 5% lactose +5% palatinit | 0.23 | 0.00 | 0.13 | 16 |
| 5% lactose +5% palatinose | 0.33 | 0.23 | 0.12 | 3 |
| 5% lactose +5% isomaltose | 2.41 | 0.00 | 0.06 | −1 |

The decreased concentrations of liberated galactose (0.13, 0.12, and 0.06 g/l) were found in all of the test solutions where palatinit, palatinose or isomaltose was added to lactose. However, the difference was little since the liberated saccharide concentration was low (0.16g /l) even in the control solution with the substrate lactose only. Since the liberated glucose concentration in the test solution did not decrease, it is concluded that no lactose inhibitory effect was appreciable in the three test samples used.

Example 4

(Confirmation of the glucoamylase activity inhibitory effect of palatinose and palatinit)

The procedures in Example 1 were repeated with the exception that palatinose and palatinit were used as a test sample and soluble starch was used as a substrate. The test samples and the substrate were dissolved in 0.1 M phosphate buffer (pH 6.8) to attain the concentration shown in Table 6 to thereby prepare two test solutions. Besides, each of the test samples or the substrate was dissolved in 0.1 M phosphate buffer (pH 6.8) to attain the concentration shown in Table 6 to thereby prepare control solutions. Each 5 ml of the aforementioned test solutions and the control solutions was subjected to the enzymatic reaction according to the same procedures as in Example 1 and the liberated glucose concentration and the decomposition inhibition ratio were measured. The results are as shown in Table 6.

[Table 6]

Table 6

| Sample | Liberated glucose concentration (g/l) | Decomposition inhibition ratio, %, against glucoamylase |
|---|---|---|
| 5% palatinit | 0.088 | |
| 5% palatinose | 0.205 | |
| 5% soluble starch | 4.657 | |
| 5% soluble starch +5% palatinit | 4.059 | 14 |
| 5% soluble starch +5% palatinose | 3.611 | 26 |

As seen in Table 6, where palatinose or palatinit was added as the test sample, the amount of glucose liberated from soluble starch was lower. Thus, these test samples have the glucoamylase activity inhibitory effect.

Example 5

(Confirmation of the sucrase activity inhibitory effect of trehalulose)

The procedures in Example 1 were repeated with the exception that trehalulose was used as a test sample and sucrose was used as a substrate. The test sample and the substrate were dissolved in 0.1 M phosphate buffer (pH 6.8) to attain the concentration shown in Table 7 to thereby prepare a test solution. Besides, the test sample or the substrate was dissolved in 0.1 M phosphate buffer (pH 6.8) to attain the concentration shown in Table 7 to thereby prepare control solutions. Each 5 ml of the aforementioned test solution and the control solutions was subjected to the enzymatic reaction according to the same procedures as in Example 1 and the liberated glucose concentration and the decomposition inhibitory ratio was determined. The results are as shown in Table 7.
[Table 7]

Table 7

| Sample | Liberated glucose concentration (g/l) | Decomposition inhibition ratio. %, against sucrose |
|---|---|---|
| 5% trehalulose | 0.558 | |
| 5% sucrose | 1.628 | |
| 5% sucrose+5% trehalulose | 1.403 | 36 |

As seen in Table 7, where trehalulose was added as a test sample, the amount of glucose liberated from sucrose decreased from 1.628 g/l to 1.403 g/l. Thus, trehalulose has the sucrase activity inhibitory effect.

[Test Example 4]

(Confirmation of the sucrase activity inhibitory effect of trehalose)

The procedures in Example 1 were repeated with the exception that trehalose was used as a test sample and sucrose was used as a substrate. Trehalose is a disaccharide where two molecules of D-glucose are bonded via their reducing residues. The test sample and the substrate were dissolved in 0.1 M phosphate buffer (pH 6.8) to attain the concentration shown in Table 8 to thereby prepare a test solution. Besides, the test sample or the substrate was dissolved in 0.1 M phosphate buffer (pH 6.8) to attain the concentration shown in Table 8 to thereby prepare control solutions. Each 5 ml of the aforementioned test solution and the control solutions was subjected to the enzymatic reaction according to the same procedures as in Example 1 and the liberated glucose concentration was measured. The results are as shown in Table 8 below.
[Table 8]

Table 8

| Sample | Liberated glucose concentration (g/l) | Decomposition inhibition ratio. %, against sucrose |
|---|---|---|
| 5% trehalose | 1.656 | |
| 5% sucrose | 1.628 | |
| 5% sucrose+5% trehalose | 1.683 | 49 |

As seen in Table 8, where trehalose was added as a test sample, the glucose concentration was 1.683 g/l, which was greater than the glucose concentration in the control solution with sucrose alone, 1.628 g/l. Thus, trehalose has substantially no sucrase activity inhibitory effect.

Example 6

(Confirmation of the glucoamylase activity inhibitory effect of trehalulose)

The procedures in Example 1 were repeated with the exception that trehalulose was used as a test sample and soluble starch was used as a substrate. The test sample and the substrate were dissolved in 0.1 M phosphate buffer (pH 6.8) to attain the concentration shown in Table 9 to thereby prepare a test solution. Besides, the test sample or the substrate was dissolved in 0.1 M phosphate buffer (pH 6.8) to attain the concentration shown in Table 9 to thereby prepare control solutions. Each 5 ml of the aforementioned test solution and the control solutions was subjected to the enzymatic reaction according to the same procedures as in Example 1 and the liberated glucose concentration and the decomposition inhibition ratio were measured. The results are as shown in Table 9.

[Table 9]

Table 9

| Sample | Liberated glucose concentration (g/l) | Decomposition inhibition ratio, %, against glucoamylase |
|---|---|---|
| 5% trehalulose | 0.411 | |
| 5% soluble starch | 2.400 | |
| 5% soluble starch +5% trehalulose | 2.028 | 28 |

As seen in Table 9, where trehalulose was added as a test sample, the glucose concentration liberated from soluble starch decreased. Thus, trehalulose has a glucoamylase activity inhibitory effect.

[Test Example 5]

(Confirmation of the glucoamylase activity inhibitory effect of trehalose)

The procedures in Example 1 were repeated with the exception that trehalose was used as a test sample and soluble starch was used as a substrate. The test sample and the substrate were dissolved in 0.1 M phosphate buffer (pH 6.8) to attain the concentration shown in Table 10 to thereby prepare a test solution. Besides, the test sample or the substrate was dissolved in 0.1 M phosphate buffer (pH 6.8) to attain the concentration shown in Table 10 to thereby prepare control solutions. Each 5 ml of the aforementioned test solution and the control solutions was subjected to the enzymatic reaction according to the same procedures as in Example 1 and the liberated glucose concentration and the decomposition inhibition ratio were measured. The results are as shown in Table 10.

[Table 10]

Table 10

| Sample | Liberated glucose concentration (g/l) | Decomposition inhibition ratio, %, against glucoamylase |
|---|---|---|
| 5% trehalose | 0.333 | |
| 5% soluble starch | 2.400 | |
| 5% soluble starch + 5% trehalose | 2.349 | 14 |

As seen in Table 10, where trehalose was added as a test sample, the glucose concentration, 2.349 g/l, was slightly less than the liberated glucose concentration in the control solution with soluble starch alone, 2.400 g/l. Thus, trehalose has a glucoamylase activity inhibitory effect, but the effect is very weak.

Example 7

(Confirmation of the sucrase activity inhibitory effect and the glucoamylase activity inhibitory effect by combinations of the test samples)

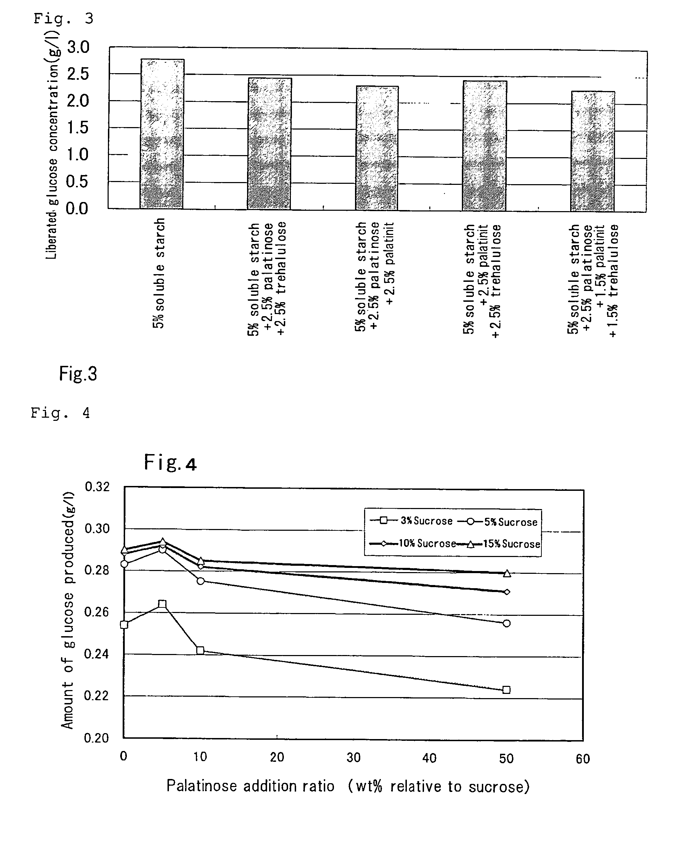

The procedures in Example 1 were repeated with the exception that combinations of palatinose, trehalulose or palatinit were used as test samples and sucrose or soluble starch was used as a substrate. Combinations of the test samples and the substrate were dissolved in 0.1 M phosphate buffer (pH 6.8) to attain the concentration shown in Fig. 2 to prepare four test solutions for the sucrase activity. Similarly, combinations of the test samples and the substrate were dissolved in 0.1 M phosphate buffer (pH 6.8) to attain the concentration shown in Fig. 3 to prepare four test solutions for the glucoamylase activity. Further, 5% sucrose, and 5% soluble starch in 0.1 M phosphate buffer (pH 6.8) were prepared as control solutions, respectively. Each 5 ml of the aforementioned test solutions and the control solutions was subjected to the enzymatic reaction according to the same procedures as in Example 1 and the liberated glucose concentration was measured. The results are as shown in Figs. 2 and 3.

As seen in Figs. 2 and 3, the inhibitory effect on the sucrase activity and the glucoamylase activity was seen also with combinations of palatinose, trehalulose and palatinit as well as with either one of them.

Example 8

Ratio of Palatinose (Pal) to the Substrate Sucrose (Suc)

1. Test Method

A. Sample Preparation

Twenty samples with the concentrations shown in Table 11, Samples A-1 through A-20, were dissolved in 0.1 M phosphate buffer (pH 6.8) to a final volume of 2.5 ml.

[Table 11]

Table 11

| A-1 | 0.5% Suc | | | [0%] * |
|-----|----------|---|-----------|--------|
| A-2 | " | + | 0.025% Pal | [5%] |
| A-3 | " | + | 0. 05 % Pal | [10%] |
| A-4 | " | + | 0.25 % Pal | [50%] |
| A-5 | 3% Suc | | | [0%] |
| A-6 | " | + | 0. 15 % Pal | [5%] |
| A-7 | " | + | 0.3 % Pal | [10%] |
| A-8 | " | + | 1.5 % Pal | [50%] |
| A-9 | 5% Suc | | | [0%] |
| A-10 | " | + | 0. 25 % Pal | [5%] |
| A-11 | " | + | 0.5 % Pal | [10%] |
| A-12 | " | + | 2.5 % Pal | [50%] |
| A-13 | 10% Suc | | | [0%] |
| A-14 | " | + | 0,5 % Pal | [5%] |
| A-15 | " | + | 1.0 % Pal | [10%] |
| A-16 | " | + | 5.0 % Pal | [50%] |
| A-17 | 15% Suc | | | [0%] |
| A-18 | " | + | 0.75% Pal | [5%] |
| A-19 | " | + | 1.5% Pal | [10%] |
| A-20 | " | + | 7.5% Pal | [50%] |
| * The percentage in square brackets indicates percentage by weight of palatinose relative to the weight of sucrose. | | | | |

B. Preparation of a Small Intestinal Enzyme Solution

This was made in the same way as in the preparation of a small intestinal enzyme solution in Example 1.

C. Measurement of the Activity

Each 0.15 ml of a small intestinal enzyme solution was added to each 2.5 ml of the aforementioned 20 samples, each mixture, and placed in a water bath at 37°C to allow reaction for 10 minutes while shaken at 60 strokes/min. At the end of the reaction, the mixture was heated in a boiling water bath for 3 min. for inactivation of the enzyme. As a blank, 0.15 ml of the intestinal enzyme solution was added to 0.1 M phosphate buffer (pH 6.8) and the enzyme was inactivated immediately.
After the reaction, the glucose concentration in each solution was measured using the F-kit Glucose (ex Roche) as in Example 1.

2. Results

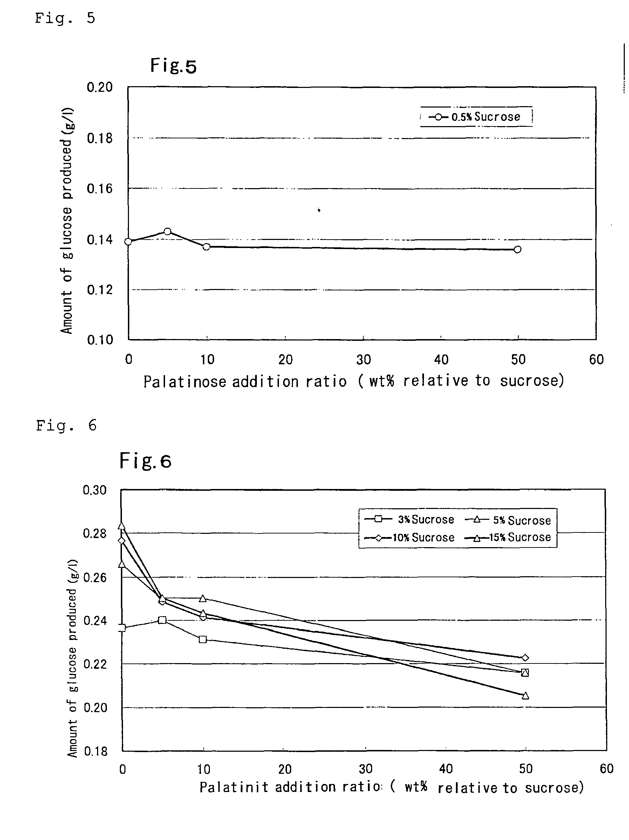

The results are as shown in Fig. 4 and Fig. 5. An inhibitory effect on the decomposition of sucrose was not clear in the case of a sucrose concentration of 0.5% (Fig. 5). However, in the cases of the sucrose concentrations of 3%, 5%, 10% and 15%, the decomposition of sucrose was suppressed when the ratio of palatinose added was 10% or greater relative to the weight of sucrose (Fig. 4). Thus, in the cases of the sucrose concentration of 3% or greater, the sucrase activity inhibition was seen when the ratio of palatinose added was 10% or greater relative to the weight of sucrose.

Example 9

Ratio of Palatinit (Nit) to the Substrate Sucrose (Suc)

1. Test Method

The procedures in Example 8 were repeated with the exception that twenty samples with the concentrations shown in Table 12, Samples B-1 through B-20, were dissolved in 0.1 M phosphate buffer (pH 6.8) to a final volume of 2.5 ml.
[Table 12]

Table 12

| B-1 | 0.5% Suc | | | [0%]* |
|---|---|---|---|---|
| B-2 | " | + | 0.025% Nit | [5%] |
| B-3 | " | + | 0.05 % Nit | [10%] |
| B-4 | " | + | 0.25 % Nit | [50%] |
| B-5 | 3% Suc | | | [0%] |
| B-6 | " | + | 0.15% Nit. | [5%] |
| B-7 | " | + | 0.3 % Nit | [10%] |
| B-8 " | " | + | 1.5 % Nit | [50%] |
| B-9 | 5% Suc | | | [0%] |
| B-10 | " | + | 0.25% Nit | [5%] |
| B-11 | " | + | 0. 5 % Nit | [10%] |
| B-12 | " | + | 2. 5 % Nit | [50%] |
| B-13 | 10% Suc | | | [0%] |
| B-14 | " | + | 0.5 % Nit | [5%] |
| B-15 | " | + | 1.0 % Nit | [10%] |
| B-16 | " | + | 5. 0 % Nit | [50%] |
| B-17 | 15% Suc | | | [0%] |
| B-18 | " | + | 0.75% Nit | [5%] |
| B-19 | " | + | 1.5 % Nit | [10%] |
| B-20 | " | + | 7.5 % Nit | [50%] |
| * The percentage in square brackets indicates percentage by weight of palatinit relative to the weight of sucrose. | | | | |

2. Results

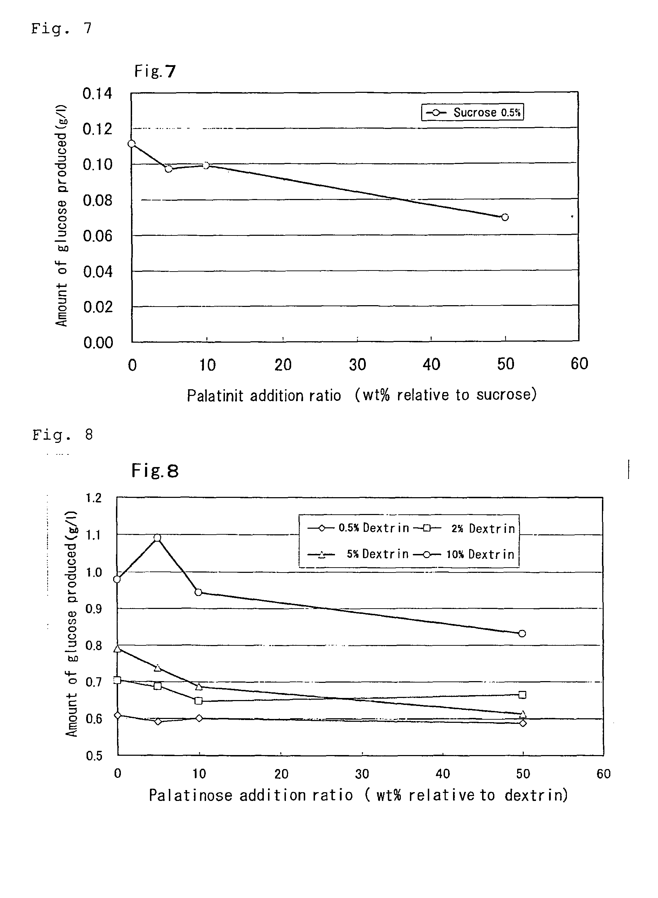

The results are as shown in Fig. 6 and Fig. 7. The inhibitory effect on the decomposition of sucrose was seen at all of the sucrose concentrations, 0.5%, 3%, 5%, 10% and 15%. The decomposition of sucrose was inhibited proportionally to the ratio of palatinit added. Therefore, palatinit seems to have a higher inhibitory effect on the decomposition of sucrose, compared to palatinose in Example 8.

Example 10

Ratio of Palatinose (Pal) to the Substrate Dextrin (Dex)

1. Test Method

The procedures in Example 8 were repeated with the exception that sixteen samples with the concentrations shown in Table 13, Samples C - 1 through C-16, were dissolved in 0.1 M phosphate buffer (pH 6.8) to a final volume of 2.5 ml.

[Table 13]

Table 13

| C-1 | 0.5% DEX | | | [0%]* |
|-----|----------|---|-----------|-------|
| C-2 | " | + | 0.025% Pal | [5%] |
| C-3 | " | + | 0. 05 % Pal | [10%] |
| C-4 | " | + | 0. 25 % Pal | [50%] |
| C-5 | 2% DEX | | | [0%] |
| C-6 | " | + | 0. 15% Pal | [5%] |
| C-7 | " | + | 0. 3 % Pa l | [10%] |
| C-8 | " | + | 1. 5 % Pal | [50%] |
| C-9 | 5% DEX | | | [0%] |
| C-10 | " | + | 0.25% Pal | [5%] |
| C-11 | " | + | 0. 5 % Pa l | [10%] |
| C-12 | " | + | 2. 5 % Pal | [50%] |
| C-13 | 10% DEX | | | [0%] |
| C-14 | " | + | 0. 5 % Pal | [5%] |
| C-15 | " | + | 1. 0 % Pal | [10%] |
| C-16 | " | + | 5. 0 % Pal | [50%] |
| * The percentage in square brackets indicates percentage by weight of palatinose relative to the weight of dextrin. | | | | |

2. Results

The results are as shown in Fig. 8. When a ratio of palatinose added was 5% relative to the weight of dextrin, no inhibitory effect on decomposition of dextrin was seen at a dextrin concentration of 10%. However, with a ratio of palatinose added above 10% relative to the weight of dextrin, the inhibitory effect on the decomposition of dextrin was seen at all of the dextrin concentrations, 0.5%, 2%, 5%, and 10%.

Example 11

Ratio of Palatinit (Nit) to the Substrate Dextrin (Dex)

1. Test Method

The procedures in Example 8 were repeated with the exception that sixteen samples with the concentrations shown in Table 14, Samples D - 1 through D-16, were dissolved in 0.1 M phosphate buffer (pH 6.8) to a final volume of 2.5 ml.

[Table 14]

Table 14

| D-1 | 0.5% DEX | | | [0%]* |
|-----|----------|---|-----------|-------|
| D-2 | " | + | 0.025% Nit | [5%] |

(continued)

| | | | | |
|---|---|---|---|---|
| D-3 | " | + | 0.05 % Nit | [10%] |
| D-4 | " | + | 0.25 % Nit | [50%] |
| D-5 | 2% DEX | | | [0%] |
| D-6 | " | + | 0.15 % Nit | [5%] |
| D-7 | " | + | 0.3 % Nit | [10%] |
| D-8 | " | + | 1. 5 % Nit | [50%] |
| D-9 | 5% DEX | | | [0%] |
| D-10 | " | + | 0.25 % Nit | [5%] |
| D-11 | " | + | 0.5% Nit | [10%] |
| D-12 | " | + | 2. 5 % Nit | [50%] |
| D-13 | 10% DEX | | | [0%] |
| D-14 | " | + | 0. 5 %Nit | [5%] |
| D-15 | " | + | 1.0 % Nit | [10%] |
| D-16 | " | + | 5. 0 %Nit | [50%] |
| * The percentage in square brackets indicates percentage by weight of palatinit relative to the weight of dextrin. | | | | |

2. Results

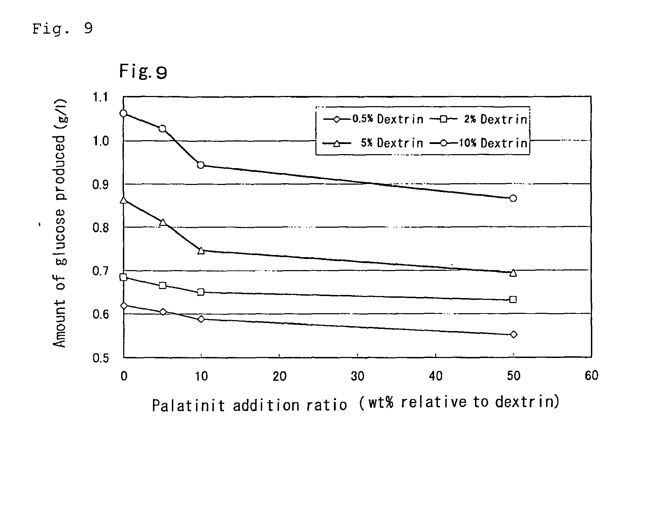

The results are as shown in Fig. 9. When a ratio of palatinit added was 5% or greater relative to the weight of dextrin, the inhibitory effect on the decomposition of dextrin was seen at all of the dextrin concentrations, 0.5%, 2%, 5%, and 10%.

Example 12

(Agent with Palatinose in a Granule Form According to the Invention)

An agent with palatinose in a granule form was prepared using crystalline palatinose in a powder form. Crystalline palatinose (trade name: Crystalline Palatinose - IC, ex Shin Mitsui Sugar Co., Ltd.) was fed at a raw material input port of a twin-screw extruder at a rate of 40 kg/hr and melted at 160 to 180°C. Subsequently, water was added at a rate of 2 kg/hr for cooling and precipitation to obtain powder. This powder was passed through sieves to obtain granules, of which 99% or more falls at 10 to 40 mesh.

Example 13

(Agent with Palatinit in a Granule Form According to the Invention)

The procedures in Example 12 were repeated to produce granules with the exception that use was made of powder palatinit (trade name: Palatinit PNM - 2, ex Shin Mitsui Sugar Co., Ltd.) instead of the crystalline palatinose.

Example 14

(Agent with Palatinose in a Powder Mixture Form According to the Invention)

An agent in a powder mixture form according to the invention was prepared with the following composition, using a universal mixer in a conventional manner.

| | |
|---|---|
| Crystalline palatinose | 85.7 wt. % |
| (Trade name: Crystalline Palatinose-IC, ex Shin Mitsui Sugar Co., Ltd.) | |
| Powdered juice | 10 wt.% |
| Anhydrous citric acid | 3 wt. % |
| Sodium citrate | 0.4 wt. % |

(continued)

| | |
|---|---|
| L-ascorbic acid | 0.5 wt. % |
| Sodium ascorbate | 0.3 wt. % |
| Riboflavin (content 10wt. %) | 0.1 wt. % |

Example 15

(Agent with Palatinit in a Powder Mixture Form According to the Invention)

The procedures in Example 14 were repeated to produce a powder mixture agent with the exception that use was made of powder palatinit (trade name: Palatinit PNM - 2, ex Shin Mitsui Sugar Co., Ltd.) instead of the crystalline palatinose.

Example 16

(Food (Fondant) with Palatinose)

Fondant was prepared with the following composition. Crystalline palatinose was fed at a raw material input port of a twin-screw extruder at a rate of 120 kg/hr and melted at 160 to 200°C. Subsequently, water was added at a rate of 5.6 kg/hr for cooling to produce microcrystals. Finally, palatinose syrup was poured at 100 kg/hr and blended with cooling.

| | |
|---|---|
| Crystalline palatinose | 120 parts by weight |
| (trade name: Crystalline Palatinose-IC, ex Shin Mitsui Sugar Co., Ltd.) | |
| Palatinose syrup | |
| (trade name: Palatinose Syrup - ISN, ex Shin Mitsui Sugar Co., Ltd.) 100 parts by weight | |

The fondant thus prepared can be used as a raw material for soft candy, or a decoration raw material for baked sweets and sweet breads.

Example 17

(Food (Fondant) with Palatinit)

The procedures in Example 16 were repeated to produce fondant with the exception that use was made of palatinit (trade name: Palatinit PNM - 2, ex Shin Mitsui Sugar Co., Ltd.) instead of the crystalline palatinose and use was made of maltitol syrup (trade name: Mabit, ex Hayashibara Shoji) instead of the palatinose syrup.

Example 18

(Food (Fondant) with both Palatinose and Trehalulose)

A fondant containing the present agent with the following composition was prepared.
Crystalline palatinose was fed at a raw material input port of a twin-screw extruder at a rate of 230 kg/hr and melted at barrel temperature of 160 to 200°C. Subsequently, water was added at a rate of 21.0 kg/hr for cooling to produce microcrystals. Finally, trehalulose syrup containing the present agent was poured at 100 kg/hr, and blended while cooled.

| | |
|---|---|
| Trehalulose syrup | 100 parts by weight |
| Crystalline palatinose | 230 parts by weight |
| (trade name: Crystalline Palatinose-IC, ex Shin Mitsui Sugar Co., Ltd.) | |

The fondant thus prepared can be used as a raw material for soft candies, or a decoration raw material for baked sweets and sweet breads.

Example 19

(Food (Fondant) with Palatinit and Trehalulose)

The procedures in Example 18 were repeated to produce a fondant with the exception that use was made of palatinit (trade name: Palatinit PNM - 2, ex Shin Mitsui Sugar Co., Ltd.) instead of the crystalline palatinose.

Example 20

(Food (Tablets) with Palatinose)

Tablets containing the present agent were prepared with the following composition. Tablets (diameter: 18 mm, thickness: 5 mm, and weight: 1.5g) were prepared by applying a compression force of 300 kg/cm$^2$ on a powder mix with the following composition.

| | |
|---|---|
| Powdered palatinose | 55 parts by weight |
| (trade name: Palatinose Powder-ICP, ex Shin Mitsui Sugar Co., Ltd.) | |
| Citric acid | 1 part by weight |
| Sugar ester | 1 part by weight |
| Aspartame | 0.05 part by weight |
| Vitamin P | 0.0002 part by weight |
| Water | 0.6 part by weight |
| Lemon flavor | Appropriate amount |

Example 21

(Food (Tablets) with Palatinit)

Tablets containing the present agent were prepared with the following composition. Tablets (diameter: 18 mm, thickness: 5 mm, and weight: 1.5g) were prepared by applying a compression force of 300 kg/cm$^2$ on a powder mix with the following composition.

| | |
|---|---|
| Palatinit | 78.0 wt. % |
| (trade name: Palatinit PNM-2, ex Shin Mitsui Sugar Co., Ltd.) | |
| Vitamin C granules | 19.5 wt. % |
| Powdered flavor | 0.4 wt. % |
| Aspartame | 0.2 wt. % |
| Lubricant | 1.9 wt. % |

Example 22

(Food (Sugar-Coated Tablets) with both Palatinose and Palatinit)

Sugar-coated tablets containing the present agent was prepared, using the agent (tablets) prepared in Example 21. The agent (tablets) prepared in Example 21 was placed in a coating pan and was soft-coated alternately with syrup having the following composition (a) and powdered palatinit at a weight ratio of 1:2 and subsequently hard-coated with the following composition (b). After the coating, the tablets were dried in air at room temperature.

(a) For soft-coating:

| | |
|---|---|
| Powdered palatinit | 62 wt. % |
| (trade name: Palatinit (type GS), ex Shin Mitsui Sugar Co., Ltd.) | |

(continued)

| | |
|---|---|
| Gum Arabic | 6.5 wt. % |
| Water | 31.5 wt. % |

(b) For hard-coating:

| | |
|---|---|
| Powdered palatinit | 65 wt. % |
| (trade name: Palatinit (type GS), ex Shin Mitsui Sugar Co., Ltd.) | |
| Gum Arabic | 3.5 wt. % |
| Water | 31.5 wt. % |
| Lemon flavor | appropriate amount |

Example 23

(Food (Drink) with Palatinose)

A drink containing the present agent was prepared, using the agent (powder mix) prepared in Example 14. The drink was prepared by dissolving 25 g of the agent prepared in Example 14 in 200 ml of hot water.

Example 24

(Food (Drink) with Trehalulose)

With the following composition a soft drink containing the present agent at a ratio as described below was prepared. The following ingredients were dissolved in 250 ml of hot water and filled in a can for 250 ml.

| | |
|---|---|
| Trehalulose syrup | 70.0 parts by weight |
| (trade name: Mildear-75, ex Shin Mitsui Sugar Co., Ltd.) | |
| Citric acid | 0.67 part by weight |
| Sodium citrate | 0.34 part by weight |
| Honey flavor | 0.25 part by weight |
| Lemon flavor | 0.014 part by weight |

Example 25

(Food (Drink) with both Palatinose and Trehalulose)

A soft drink containing the present agent was prepared with the following composition. The following ingredients were dissolved at the following concentration in hot water to a total of 250 g, and filled in a drink can for 250 ml.

| | |
|---|---|
| Crystalline palatinose | 4 parts by weight |
| (trade name: Crystalline Palatinose - IC, ex Shin Mitsui Sugar Co., Ltd.) | |
| Trehalulose syrup | 6 parts by weight |
| (Trade name: Mildear - 75, ex Shin Mitsui Sugar Co., Ltd.) | |
| Citric acid | 0.15 part by weight |
| Vitamin C | 0.03 part by weight |
| Sodium chloride | 0.05 part by weight |
| Potassium chloride | 0.04 part by weight |
| Calcium chloride | 0.012 part by weight |
| Magnesium carbonate | 0.002 part by weight |

(continued)

| Sodium glutamate | 0.006 part by weight |
|---|---|
| Stevia | 0.01 part by weight |
| Vitamin P | 0.0004 part by weight |
| Flavor | appropriate amount |

Example 26

(Food (Sports Drink) with Trehalulose)

A sports drink containing the present agent was prepared with the following proportion. The following ingredients were dissolved in 215 ml of hot water, and filled in a drink can for 250 ml.

| Trehalulose syrup | 50 parts by weight |
|---|---|
| (trade name: Mildear-75 with a Trehalulose content of 83 to 89%, ex Shin Mitsui Sugar Co., Ltd.) | |
| Vitamin C | 0.075 part by weight |
| Vitamin B1 hydrochloride | 0.005 part by weight |
| Sodium citrate | 0.255 part by weight |
| Magnesium chloride | 0.03 part by weight |
| Calcium lactate | 0.03 part by weight |
| Anhydrous citric acid | 0.36 part by weight |
| Flavor | 0.03 part by weight |

Example 27

(Food (Candy) with Trehalulose)

Hard candies containing the present agent were prepared with the following composition. Trehalulose syrup was added to a dissolution vessel and stirred with heating. Subsequently, the solution was heated *in vacuo* up to a temperature of 120°C at a pressure of 86.7 KPa Gauge (vacuum 650 mmHg) and, then, citric acid, aspartame, tartaric acid, Red Color, Blue Color and grape flavor were added. After cooled to approximately 70 to 80°C, the mixture was molded into candies of each 4g, which was each packaged.

| Trehalulose syrup | 100 parts by weight |
|---|---|
| (trade name: Mildear - 75, 89%, ex Shin Mitsui Sugar | trehalulose content of 83 to Co., Ltd.) |
| Grape flavor | 0.25 part by weight |
| (No.6 - 6240, ex Hasegawa Flavoring Corporation) | |
| Red Color | 0.10 part by weight |
| (TH - L, ex Hasegawa Flavoring Corporation) | |
| Blue Color | 0.05 part by weight |
| (TH - 3L, ex Hasegawa Flavoring Corporation) | |
| Citric acid | 1.00 part by weight |
| Tartaric acid | 0.30 part by weight |
| Aspartame | 0.12 part by weight |

Example 28

(Food (Candy) with both Palatinose and Trehalulose)

Hard candies containing the present agent were prepared with the following composition. First, crystalline palatinose, trehalulose syrup and water were added to a dissolution vessel and stirred to dissolve with heating. Subsequently, the solution was heated *in vacuo* up to a temperature of 120°C at a pressure of 700 mmHg and, then, citric acid, aspartame, Vitamin P and lemon flavor were added. After cooled to approximately 70 to 80°C, the mixture was molded into candies of each 4g, which was each packaged.

| Crystalline palatinose | 70 parts by weight |
|---|---|
| (trade name: Crystalline Palatinose - IC, ex Shin Mitsui Sugar Co., Ltd.) | |
| Trehalulose syrup | 40 parts by weight |
| (trade name: Mildear - 75, ex Shin Mitsui Sugar Co., Ltd.) | |
| Citric acid | 2 parts by weight |
| Aspartame | 0.07 part by weight |
| Vitamin P | 0.003 part by weight |
| Water | 15 parts by weight |
| Lemon flavor | appropriate amount |

Example 29

(Food (Jelly drink) with Trehalulose)

A jelly drink with orange taste containing the present agent was prepared with the following composition. First, palatinose syrup and water were mixed with each other to which a gelling agent was, subsequently, added portion-wise to dissolve while heated to 90°C. After cooled to 70°C, the remaining ingredients were added and stirred to dissolve. The solution was filled in a cheer pack. After sealed, the package was sterilized at 90°C for 20 min. and then cooled.

| Palatinose syrup (Bx. 75) | 15 parts by weight |
|---|---|
| (trade name: Palatinose Syrup -TN, ex Shin Mitsui Sugar Co., Ltd.) | |
| Gelling agent | 1 part by weight |
| 1/5 concentrated orange juice | 4 parts by weight |
| Water | 80 parts by weight |
| Citric acid | 0.35 part by weight |
| Sodium citrate | 0.2 part by weight |
| Vitamin C | 0.6 part by weight |
| β-carotene | 0.01 part by weight |
| Stevia | 0.01 part by weight |
| Vitamin P | 0.0004 part by weight |
| Orange flavor | appropriate amount |

Example 30

(Food (Jelly drink) with both Palatinose and Trehalulose)

A jelly drink with orange taste containing the present agent was prepared with the following composition. First, palatinose, trehalulose syrup and water were mixed together and a gelling agent was added portion-wise to dissolve while heated to 90°C. Subsequently, after cooled to 70°C, the remaining ingredients were added and dissolved by stirring. The solution was filled in a cheer pack and, after sealed, sterilized at 90°C for 20 minutes and then cooled.

| Crystalline palatinose | 5 parts by weight |
|---|---|
| (trade name: Crystalline Palatinose - IC, ex Shin Mitsui Sugar Co., Ltd.) | |
| Trehalulose syrup | 10 parts by weight |
| (trade name: Palatinose Syrup -TN, ex Shin Mitsui Sugar Co., Ltd.) | |
| Gelling agent | 1 part by weight |
| 1/5 concentrated orange juice | 4 parts by weight |
| Water | 80 parts by weight |
| Citric acid | 0.35 part by weight |
| Sodium citrate | 0.20 part by weight |
| Vitamin C | 0.6 part by weight |
| β-carotene | 0.01 part by weight |

(continued)

| | |
|---|---|
| Stevia | 0.01 part by weight |
| Vitamin P | 0.0004 part by weight |
| Orange flavor | appropriate amount |

Example 31

(Food (Pancake) with Palatinose)

Pancakes containing the present agent were prepared with the following composition. First, wheat flour, baking powder and powdered palatinose were combined and sieved. Milk and eggs were combined and stirred well, to which the sieved powder mix was added and the mixture was briefly blended to become uniform with a whip to obtain a batter. The batter was placed in a hot plate at 200°C. After it became golden and bubbles were seen on the upper surface, it was turned over. When the other side became golden, the pancake was taken out from the plate, on which butter and maple syrup were then placed.

| | |
|---|---|
| Wheat flour | 200 g |
| Baking powder | 6 g |
| Powdered palatinose | 70 g |
| (trade name: Powdered Palatinose - ICP, ex Shin Mitsui Sugar Co., Ltd.) | |
| Milk | 180 ml |
| Eggs | 50 g |
| Water | 45 ml |
| Butter | 10 g |
| Maple syrup | 15 g |

Example 32

(Food (Pancake) with Trehalulose)

Pancakes containing the present agent were prepared with the following composition. First, wheat flour and baking powder were combined and sieved. Eggs and trehalulose syrup were combined and stirred well, to which milk was added and stirred well and the sieved powder mix was added. The mixture was briefly blended to become uniform with a whip to obtain a batter. The batter was placed in a hot plate at 200°C. After it became golden and bubbles were seen on the upper surface, it was turned over. When the other side became golden, the pancake was taken out from the plate, on which butter and maple syrup were then placed.

| | |
|---|---|
| Wheat flour | 200 g |
| Baking powder | 6 g |
| Milk | 150 ml |
| Eggs | 50 g |
| Trehalulose syrup | 90 g |
| (trade name: Mildear - 75, ex Shin Mitsui Sugar Co., Ltd.) | |
| Water | 45 ml |
| Butter | 10 g |
| Maple syrup | 15 g |

Example 33

(Sucrose-based sugar stick containing palatinose or palatinit)

One thousand grams of sucrose was mixed with 100 g of palatinose (trade name: Crystalline Palatinose - IC, ex Shin Mitsui Sugar Co., Ltd.) or palatinit (trade name: Palatinit PN, ex Shin Mitsui Sugar Co., Ltd.) and filled in a stick package in an amount of 5 g per stick. When the stick sugar is dissolved in 150 ml of coffee or tea, provided is a drink containing 3.0% of sucrose and 0.3% of palatinose or palatinit.

Example 34

(Portion syrup containing sucrose and trehalulose)

Five hundred grams of sucrose was mixed with 65 g of Mildear - 85 (containing 50 g of trehalulose, ex Shin Mitsui Sugar, Co., Ltd.,) and 175 g of water and processed into portion syrups of 7.4 g per portion. When the portion syrup is dissolved in 165 ml of iced coffee or iced tea, provided is a drink containing 3% of sucrose and 0.3% of trehalulose.

Example 35

(Sucrose-based drinks containing palatinose, trehalulose or palatinit)

Drinks were prepared with the compositions shown in the following Table 15.
[Table 15]

Table 15

| 1. Use of palatinose | | 2. Use of trehalulose | | 3. Use of palatinit | |
|---|---|---|---|---|---|
| Ingredient | Per can | Ingredient | Per can | Ingredient | Per can |
| Sucrose | 10.5 g | Sucrose | 10.5 g | Sucrose | 10.5 g |
| Palatinose (Crystalline Palatinose IC*) | 1.05 g | Trehalulose syrup (Mildear-75*) | 1.56 g | Palatinit (Palatinit PN*) | 1.05 g |
| 1/5 Concentrated orange juice | 2 g | 1/5 Concentrated orange juice | 2g | 1/5 Concentrated orange juice | 2 g |
| Citric acid | 0.35 g | Citric acid | 0.35 g | Citric acid | 0.35 g |
| Vitamin C | 0.6 g | Vitamin C | 0.6 g | Vitamin C | 0.6 g |
| Stevia | 0.08 g | Stevia | 0.08 g | Stevia | 0.08 g |
| Orange flavor | Appropriate amount | Orange flavor | Appropriate amount | Orange flavor | Appropriate amount |
| Water | 335.42 g | Water | 334.91 g | Water | 335.42 g |
| Total weight | 350 g | Total weight | 350 g | Total weight | 350 g |
| * ex Mitsui Sugar Co., Ltd. | | | | | |

Example 36

(Dried soup)

Dried soup was prepared with the compositions shown in Table 16.
[Table 16]

Table 16

| 1. Use of palatinose | | 2. Use of trehalulose | | 3. Use of palatinit | |
|---|---|---|---|---|---|
| Ingredient | Composition (Ratio by weight) | Ingredient | Composition (Ratio by weight) | Ingredient | Composition (Ratio by weight) |
| Whole milk powder | 30 | Whole milk powder | 30 | Whole milk powder | 30 |
| Edible plant oil | 20 | Edible plant oil | 20 | Edible plant oil | 20 |
| Powdered vegetables (corn, onion) | 18 | Powdered vegetables (corn, onion) | 18 | Powdered vegetables (corn, onion) | 18 |
| Milk protein | 12 | Milk protein | 12 | Milk protein | 12 |
| Salt | 7 | Salt | 7 | Salt | 7 |
| Seasoning (Sun-Like Taste Base**) | 5 | Seasoning (Sun-Like Taste Base**) | 5 | Seasoning (Sun-Like Taste Base**) | 5 |
| Yeast extract | 4 | Yeast extract | 4 | Yeast extract | 4 |
| Dextrin | 2 | Dextrin | 2 | Dextrin | 2 |
| Spice | 1 | Spice | 1 | Spice | 1 |
| Thickening agent (guar gum) | 0.7 | Thickening agent (guar gum) | 0.6 | Thickening agent (guar gum) | 0.7 |
| Palatinose * (Crystalline Palatinose IC*) | 0.2 | Trehalulose (Mildear -75*) | 0.3 | Palatinit (Palatinit PN*) | 0.2 |
| Intensity sweetener (Stevia) | 0.1 | Intensity sweetener (Stevia) | 0.1 | Intensity sweetener (Stevia) | 0.1 |
| Total | 100 | Total | 100 | Total | 100 |
| * ex Mitsui Sugar Co., Ltd.<br>** ex San-Ei Gen F.F.L. Inc. | | | | | |

Example 37

[(Dressings)

Dressings were prepared with the compositions shown in Table 17.
[Table 17]

Table 17

| 1. Use of palatinose | | 2. Use of trehalulose | | 3. Use of palatinit | |
|---|---|---|---|---|---|
| Ingredient | Composition (Ratio by weight) | Ingredient | Composition (Ratio by weight) | Ingredient | Composition (Ratio by weight) |
| Edible plant oil | 48 | Edible plant oil | 48 | Edible plant oil | 48 |
| Vinegar | 28 | Vinegar | 28 | Vinegar | 28 |
| Onion | 12 | Onion | 12 | Onion | 12 |
| Seasoning (Sun-Like Taste Base**) | 3.6 | Seasoning (Sun-Like Taste Base**) | 3.5 | Seasoning (Sun-Like Taste Base**) | 3.6 |
| Salt | 3 | Salt | 3 | Salt | 3 |
| Spice | 3 | Spice | 3 | Spice | 3 |
| Dextrin | 2 | Dextrin | 2 | Dextrin | 2 |
| Palatinose (Crystalline Palatinose IC*) | 0.2 | Trehalulose (Mildear -75*) | 0.3 | Palatinit (Palatinit PN*) | 0.2 |
| Emulsifier | 0.1 | Emulsifier | 0.1 | Emulsifier | 0.1 |
| Intensity sweetener (Stevia) | 0.1 | Intensity sweetener (Stevia) | 0.1 | Intensity sweetener (Stevia) | 0.1 |
| Total | 100 | Total | 100 | Total | 100 |

\* ex Mitsui Sugar Co., Ltd.
\*\* ex San-Ei Gen F.F.I., Inc.

Example 38

(Feeds with palatinose)

Feeds comprising the present agent were prepared according to the compositions shown in Table 18. The standard composition there is a reference and the other compositions are examples of the present invention. The compositions are expressed in wt. %.
[Table 18]

Table18

| Ingredient | Palatinose added | Trehalulose added | Palatinit added | Palatinose /trehalulose added | Palatinose /palatinit added | Trehalulose /palatinit added | Standard (Reference) |
|---|---|---|---|---|---|---|---|
| Corn starch | 28 | 28 | 43 | 28 | 28 | 28 | 48 |
| Sucrose | 7 | 7 | 7 | 7 | 7 | 7 | 7 |

(continued)

| Ingredient | Palatinose added | Trehalulose added | Palatinit added | Palatinose /trehalulose added | Palatinose /palatinit added | Trehalulose /palatinit added | Standard (Reference) |
|---|---|---|---|---|---|---|---|
| Palatinose (tradename: Crystalline Palatinose-IC Shin Mitsui Sugar. Co.. Ltd.) | 20 | - | - | 10 | 15 | - | - |
| Trehalulose (tradename: Mildear -75 Shin Mitsui Sugar, Co., Ltd.) | - | 20 | - | 10 | - | 15 | - |
| Palatinit (tradename: Palatinit PNM-2 Shin Mitsui Sugar. Co., Ltd.) | - | - | 5 | - | 5 | 5 | - |
| Skim milk | 28 | 28 | 28 | 28 | 28 | 28 | 28 |
| Wheat flour | 6 | 6 | 6 | 6 | 6 | 6 | 6 |
| Yeast | 4 | 4 | 4 | 4 | 4 | 4 | 4 |
| Alfalfa | 3 | 3 | 3 | 3 | 3 | 3 | 3 |
| Salt | 3 | 3 | 3 | 3 | 3 | 3 | 3 |
| Liver extract powder | 1 | 1 | 1 | 1 | 1 | 1 | 1 |

Example 39

(Sucrose-based raw material for mixed feeds)

Raw materials for mixed feeds were prepared with the compositions shown in Table 19.
[Table 19]

Table 19

| 1. Use of palatinose | | 2. Use of trehalulose | | 3. Use of palatinit | |
|---|---|---|---|---|---|
| Ingredient | Ratio by weight | Ingredient | Ratio by weight | Ingredient | Ratio by weight |
| Sucrose | 100 | Sucrose | 100 | Sucrose | 100 |
| Palatinose (Crystalline Palatinose IC*) | 10 | Trehalulose syrup (Mildear-75*) | 14.8 | Palatinit (Palatinit PN*) | 10 |
| Water | 47 | Water | 42.3 | Water | 47 |
| * ex Mitsui Sugar Co., Ltd. | | | | | |

The sucrose-based raw materials for mixed feeds are in a syrup form and is mixed with other raw materials for feeds before use. The other raw materials for feeds can include dried plants and dried proteins which do not contain sucrose, starch or dextrin. When 47.1 g of the sucrose-based raw material for mixed feeds are mixed with 1000 g of other raw material for feeds, prepared is a feed comprising at 3% of sucrose and 0.3% of the present inhibitor.

Example 40

(Dextrin-based mixed feeds)

Raw materials were mixed in the compositions shown in Table 20 and the mixture was dried in a fluidized bed dryer to prepare granulated feeds.
[Table 20]

Table 20

| 1. Use of palatinose | | 2. Use of trehalulose | | 3. Use of palatinit | |
|---|---|---|---|---|---|
| Ingredient | Ratio by weight | Ingredient | Ratio by weight | Ingredient | Ratio by weight |
| Dextrin | 100 | Dextrin | 100 | Dextrin | 100 |
| Palatinose (Crystalline Palatinose IC*) | 10 | Trehalulose syrup (Mildear-75*) | 14.8 | Palatinit (Palatinit PN*) | 10 |
| Water | 10 | Water | 10 | Water | 10 |
| * ex Mitsui Sugar Co., Ltd. | | | | | |

The granulated feeds are mixed with other raw materials for feeds before use. The other feed raw materials can include dried plants and dried proteins which do not contain sucrose, starch or dextrin. When 22.7 g of the granulated feed are mixed with 1000 g of other raw materials for feed, provided is a feed comprising 2% of dextrin and 0.2% of the present inhibitor.

Brief Description on the Drawings

Fig. 1 is a graph showing the intensity of the sucrase activity inhibition by palatinose.
Fig. 2 is a graph showing the sucrase activity inhibitory effect by the combination of the test samples.
Fig. 3 is a graph showing the glucoamylase activity inhibitory effect by the combination of the test samples.
Fig. 4 is a graph showing the sucrase activity inhibitory effect at the various sucrose concentrations and the various palatinose addition ratios relative to the sucrose.

Fig. 5 is a graph showing the sucrase activity inhibitory effect at the various sucrose concentrations and the various palatinose addition ratios relative to the sucrose.

Fig. 6 is a graph showing the sucrase activity inhibitory effect at the various sucrose concentrations and the various palatinit addition ratios relative to the sucrose.

Fig. 7 is a graph showing the sucrase activity inhibitory effect at the various sucrose concentrations and the various palatinit addition ratios relative to the sucrose.

Fig. 8 is a graph showing the glucoamylase activity inhibitory effect at the various dextrin concentrations and the various palatinose addition ratios relative to the dextrin.

Fig. 9 is a graph showing the glucoamylase activity inhibitory effect at the various dextrin concentrations and the various palatinit addition ratios relative to the dextrin.

**Claims**

1. A sucrase activity inhibitor comprising at least one saccharide selected from the group consisting of palatinose, trehalulose and palatinit.

2. A glucoamylase activity inhibitor comprising at least one saccharide selected from the group consisting of palatinose, trehalulose and palatinit.

3. A food comprising at least one of the inhibitors described in Claim 1 and Claim 2.

4. A feed comprising at least one of the inhibitors described in Claim 1 and Claim 2.

5. A food or feed comprising the inhibitor described in Claim 1 and sucrose.

6. A food or feed comprising the inhibitor described in Claim 2 and starch.

7. A food or feed comprising the inhibitor described in Claim 2 and at least one of starch and dextrin.

8. A food or feed comprising at least 3 wt. % of sucrose and at least 10 wt. %, relative to weight of the sucrose, of the sucrase activity inhibitor described in Claim 1.

9. A food or feed comprising at least 2 wt. % of at least one of starch and dextrin and at least 10 wt. %, relative to weight of the starch and dextrin, of the glucoamylase activity inhibitor described in Claim 2.

Fig. 1

Fig.1

Fig. 2

Fig.2

Fig. 3

Fig.3

Fig. 4

Fig. 5

Fig.5

Amount of glucose produced (g/l)

0.20

0.18

0.16

0.14

0.12

0.10

—○— 0.5% Sucrose

0    10    20    30    40    50    60

Palatinose addition ratio ( wt% relative to sucrose)

Fig. 6

Fig.6

Amount of glucose produced (g/l)

0.30

0.28

0.26

0.24

0.22

0.20

0.18

—□— 3% Sucrose   —△— 5% Sucrose
—◇—10% Sucrose   —△—15% Sucrose

0    10    20    30    40    50    60

Palatinit addition ratio ( wt% relative to sucrose)

Fig. 7

Fig.7

Fig. 8

Fig.8

EP 1 864 670 A1

Fig. 9

Fig.9

35

# EP 1 864 670 A1

<table>
<tr><td colspan="2" align="center">INTERNATIONAL SEARCH REPORT</td><td>International application No.<br>PCT/JP2006/306261</td></tr>
</table>

A.  CLASSIFICATION OF SUBJECT MATTER
**A61K31/7016**(2006.01), **A61P1/14**(2006.01), **A61P3/04**(2006.01), **A61P3/06**
(2006.01), **A61P3/10**(2006.01), **A61P43/00**(2006.01), **A23L1/30**(2006.01),
**C07H3/04**(2006.01)
According to International Patent Classification (IPC) or to both national classification and IPC

B.  FIELDS SEARCHED

Minimum documentation searched (classification system followed by classification symbols)
A23L1/30, A61K31/7016, A61P1/14, A61P3/04, A61P3/06, A61P3/10, A61P43/00,
C07H3/04

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
FSTA(STN), CAplus(STN), MEDLINE(STN)

C.  DOCUMENTS CONSIDERED TO BE RELEVANT

| Category* | Citation of document, with indication, where appropriate, of the relevant passages | Relevant to claim No. |
|---|---|---|
| X | JP 2004-315499 A  (SHIN MITSUI SUGAR CO., LTD.),<br>11 November, 2004 (11.11.04),<br>Full text; particularly, Claims; examples<br>& WO 2004/045312 A1 | 1-9 |
| A | JP 2001-521900 A  (SUEDZUCKER AG.),<br>13 November, 2001 (13.11.01),<br>& WO 99/22740 A1 | 1-9 |
| A | Kazuhiko YAMADA et al., Inhibitory effects of<br>D-tagatose on small intestinal disaccharidase<br>activity in the rat, Shoka to Kyushu, 2002,<br>Volume Date 2001, 24(2), 61-64 | 1-9 |

☒  Further documents are listed in the continuation of Box C.      ☐  See patent family annex.

| | |
|---|---|
| * Special categories of cited documents:<br>"A" document defining the general state of the art which is not considered   to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>12 July, 2006 (12.07.06) | Date of mailing of the international search report<br>18 July, 2006 (18.07.06) |
|---|---|
| Name and mailing address of the ISA/<br>Japanese Patent Office | Authorized officer |
| Facsimile No. | Telephone No. |

Form PCT/ISA/210 (second sheet) (April 2005)

**EP 1 864 670 A1**

**INTERNATIONAL SEARCH REPORT**

| International application No. |
| --- |
| PCT/JP2006/306261 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | Toshinao GODA et al., Hydrolysis of palatinose by rat intestinal sucrase-isomaltase complex, Nippon Eiyo, Shokuryo Gakkaishi, 1983, 36(3), 169-173 | 1-9 |

Form PCT/ISA/210 (continuation of second sheet) (April 2005)

37

**INTERNATIONAL SEARCH REPORT**

| International application No. |
| --- |
| PCT/JP2006/306261 |

---

**Box No. II**  **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III**  **Observations where unity of invention is lacking (Continuation of item 3 of first sheet)**

This International Searching Authority found multiple inventions in this international application, as follows:
   Any of the sugars and sugar alcohols described in the inventions according to claims 1-9 is well known as, for example, a sweetener, and the matter that these components are contained cannot be considered as a special technical feature, and enzymes to be inhibited by the sucrase activity inhibitor and a glucoamylase activity inhibitor are different. Further, in this application, an invention relating to a pharmaceutical and an invention relating to a food and a feed are included, however, both are different in their technical fields, and it should be understood that their special technical features are different.
   Such being the case, in this application, (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the
☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

| **INTERNATIONAL SEARCH REPORT** | International application No. |
|---|---|
| | PCT/JP2006/306261 |

Continuation of Box No. III of continuation of first sheet (2)

    according to the combination of the compound and the use, at least 6 inventions that are not considered to be so linked as to form a single general inventive concept are included.

Form PCT/ISA/210 (extra sheet) (April 2005)

**INTERNATIONAL SEARCH REPORT**

International application No.

PCT/JP2006/306261

All the sugars and sugar alcohols described in claims 1-9 are well known as, for example, a sweetener, and a food or a feed containing any of these saccharides is well known without showing any document, and the invention of a pharmaceutical of claims 1-2 and the invention of a food or a feed of claims 3-9 do not satisfy the requirement of unity, therefore, for the claims, a search was completed at the time when a document that defeats novelty of claims 8-9 was found.

Further, a search regarding a sucrase inhibitory action or a glucoamylase inhibitory action per se of each saccharide of claims 1-2 was completely performed, however, a search regarding a specific medicinal use based on the inhibitory actions was completed at the time when a document that defeats novelty of claims 1-2 was found because there is no unity between the inventions of pharmaceuticals containing the respective sugar compounds.

**EP 1 864 670 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9412057 A
- JP HEI6065080 A
- JP HEI8023973 A
- JP HEI11286449 A
- JP 2004352649 A
- JP 2002012547 A
- JP 2002255806 A
- JP 2004113068 A
- JP SHO56125398 A
- JP SHO54092909 A

**Non-patent literature cited in the description**

- **B. L. NICHOLS ; S. AVERY ; P. SEN ; D. M. SWALLOW ; D. HAHN ; E. STERCHI.** *Proceedings of the National Academy of Sciences USA,* 2003, vol. 100 (3), 1432-1437
- **T. GODA ; N. HOSOYA.** *Nippon Eiyo Shokuryo Gakkaishi (in Japanese) (Journal of Japanese Society of Nutrition and Food Science,* 1983, vol. 36 (3), 169-173
- **K. YAMADA ; H. SHINOHARA ; N. HOSOYA.** *Nutrition Reports International,* 1985, vol. 32 (5), 1211-1220
- **S.C. ZIESENITZ.** *Zeitschrift fur Ernahrungswissenshaft,* 1986, vol. 25, 253-258
- **AKIKAZU TAKEDA ; HITOSHI HASHIMOTO ; HIROSHI ITO.** Satou-hyakka. Sugar Industry Association inc. and the Japan Sugar Refiners' Association, 11-12